(19) Europäisches Patentamt
European Patent Office
Office européen des brevets

(11) **EP 2 405 671 A1**

(12) **EUROPEAN PATENT APPLICATION**
published in accordance with Art. 153(4) EPC

(43) Date of publication:
**11.01.2012 Bulletin 2012/02**

(21) Application number: **10748520.3**

(22) Date of filing: **03.03.2010**

(51) Int Cl.:
**H04R 1/34** (2006.01)   **A61B 8/00** (2006.01)
**H04R 17/00** (2006.01)

(86) International application number:
**PCT/JP2010/001469**

(87) International publication number:
**WO 2010/100921 (10.09.2010 Gazette 2010/36)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **04.03.2009 JP 2009051314**
**04.03.2009 JP 2009051315**
**09.03.2009 JP 2009054551**

(71) Applicant: **Panasonic Corporation**
**Kadoma-shi**
**Osaka 571-8501 (JP)**

(72) Inventor: **SAITO, Koetsu**
**2-1-61, Shiromi,Chuo-ku, Osaka 540-6207 (JP)**

(74) Representative: **Grünecker, Kinkeldey,**
**Stockmair & Schwanhäusser**
**Anwaltssozietät**
**Leopoldstrasse 4**
**80802 München (DE)**

(54) **ULTRASONIC TRANSDUCER, ULTRASONIC PROBE, AND ULTRASONIC DIAGNOSTIC DEVICE**

(57)   Provided are an ultrasonic transducer, an ultrasonic probe, and an ultrasonic diagnostic device which enable an ultrasonic beam in the depth direction of diagnosis to be narrowly focused in a wide region, thereby obtaining a high-resolution diagnostic image. An ultrasonic transducer (100) is provided with an ultrasonic vibrator (110), a shape change layer (120) which is provided on the subject side of the ultrasonic vibrator and the shape of which is changed by an electrical signal, electrodes (141, 142) which are respectively disposed on both surfaces of the shape change layer (120), and a variable power source (140) which applies the electrical signal between the electrodes (141, 142), wherein the variable power source (140) varies the focus or diffusion of the ultrasonic beam by changing the shape of the shape change layer (120); by controlling the electrical signal to be applied between the electrodes (141, 142).

FIG.3

**Description**

Technical Field

The present invention relates to an ultrasonic transducer, an ultrasonic probe and an ultrasonic diagnostic apparatus that are used to obtain diagnostic information of a subject by applying ultrasonic waves to the subject, such as an organism, so as to transmit and receive ultrasonic waves to and from the subject.

Background Art

The ultrasonic diagnostic apparatus applies ultrasonic waves into a subject, such as an organism of a human being, an animal, or the like, and by detecting echo signals reflected by the inside of the organism, a tomographic image or the like of a tissue inside the organism is displayed on a monitor so that information required for diagnosis of the subject is provided. In this case, the ultrasonic diagnostic apparatus utilizes an ultrasonic transducer and an ultrasonic probe so as to transmit ultrasonic waves into the subject and receive echo signals from the inside of the subject.

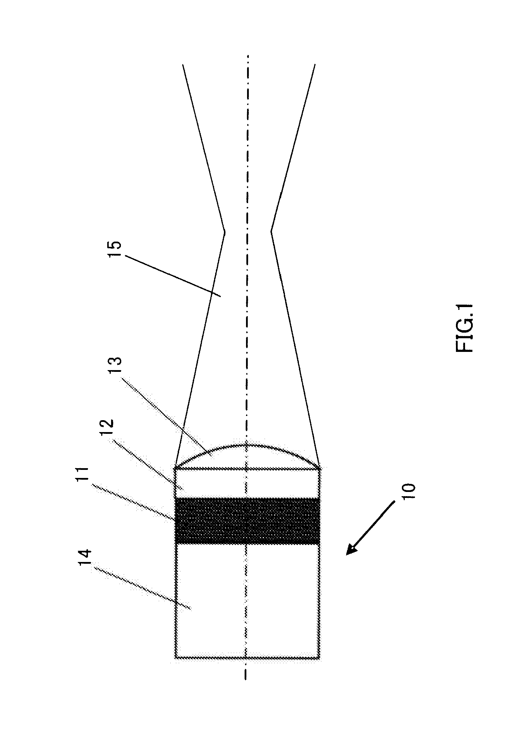

FIG.1 is a schematic view that shows a structure of an ultrasonic transducer.

As shown in FIG.1, ultrasonic transducer 10 transmits and receives ultrasonic waves to and from a subject (not shown). Ultrasonic transducer 10 is provided with ultrasonic resonator 11, one or more acoustic matching layers 12 (one layer in FIG.1) that are formed on the front face (right direction in FIG.1) on the subject side of ultrasonic resonator 11, acoustic lens 13 that is attached to the subject side surface of acoustic matching layer 12, and backing material 14 that is installed on the back face corresponding to the side opposite to acoustic matching layer 12 relative to ultrasonic resonator 11.

Electrodes (not shown) are respectively disposed on the front face and back face of ultrasonic resonator 11, and electrical signals are transmitted and received to and from ultrasonic resonator 11.

Ultrasonic resonator 11 is made of a material such as a piezoelectric ceramic material of PZT (Lead Zirconate Titanate) based material, a single crystal, a composite piezoelectric material that is a composite material between the above-mentioned material and a polymer, a polymer piezoelectric material typically represented by, for example, PVDF (PolyVinylidine DiFluoride), or the like, and converts a voltage into ultrasonic waves, and transmits them into a subject, or converts echoes reflected from the inside of the subject into electrical signals so as to be received.

Acoustic matching layer 12 is installed so as to effectively transmit and receive ultrasonic waves to and from the inside of a subject.

Backing material 14 is combined with ultrasonic resonator 11 so as to hold this, and has a function for attenuating unnecessary ultrasonic waves.

Acoustic lens 13 is installed so as to focus an ultrasonic beam and consequently to improve the resolution of a tomographic image, and the focus for ultrasonic beam 15 of this acoustic lens 13 is fixed, and the focus is made at a distance of virtually one point.

On the other hand, as a focusing method for an ultrasonic beam, an arrangement in which the focusing position is variably changed has been known, and such an arrangement has also been proposed in which voids are formed on the two ends of an acoustic lens, with a liquid being allowed to flow through the voids, and by adjusting the amount of the liquid, the curvature radius of the acoustic lens is variably changed (for example, see Patent Literature 1).

Moreover, another structure has been known in which a piezoelectric bimorph or a shape-memory alloy is formed on the backface side of an ultrasonic resonator so that the curvature of the ultrasonic resonator is variably changed and the focusing beam is consequently variably changed (for example, see Patent Literatures 2 and 3).

Furthermore, still another structure has been known in which a material that allows the acoustic velocity to be variably changed is installed between the ultrasonic resonator and a subject so that by variably changing the acoustic velocity, the focus of an ultrasonic beam is consequently variably changed (for example, see Patent Literature 4).

In recent years, an ultrasonic diagnostic apparatus has been proposed in which, by carrying out a three-dimensional scanning process with an ultrasonic beam direction being variably changed, ultrasonic waves from the inside of a subject are three-dimensionally received so that a three-dimensional image is displayed so as to execute an image diagnosis.

As one example of such an apparatus, a system has been proposed in which an ultrasonic transducer in which a plurality of ultrasonic resonators are one-dimensionally aligned is mechanically moved or rocked reciprocally in a direction orthogonal to the aligning direction by using a driving system such as a motor so as to carry out a scanning process so that three-dimensional image data are obtained.

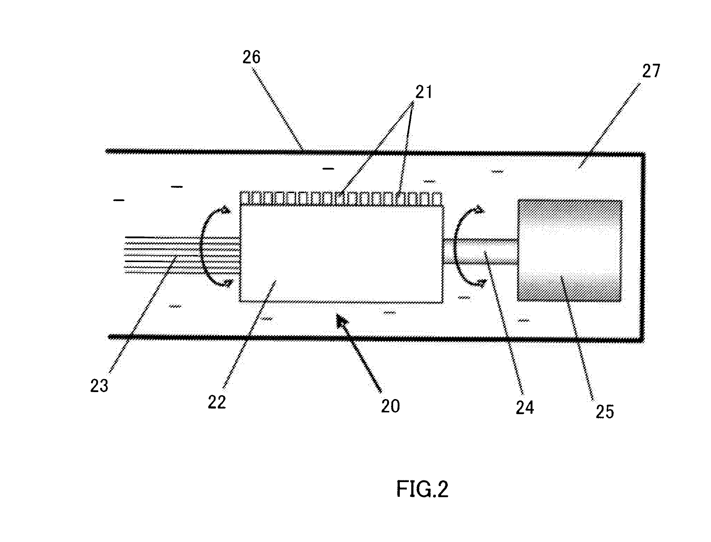

FIG.2 is a schematic view that shows a structure of an ultrasonic transducer of a type referred to as an electron scanning type.

As shown in FIG.2, ultrasonic transducer 20 is provided with ultrasonic resonators 21 that transmit and receive ultrasonic waves. A plurality of ultrasonic resonators 21 are arranged on holding base 22 such as a backing material. Ultrasonic transducer 20 is held by and joined to acoustic window 26, and the inside of acoustic window 26 is filled with ultrasonic wave propagating medium 27 that is a liquid.

Ultrasonic resonators 21 thus arranged are classified into desired groups, and a driving signal is input to each of ultrasonic resonators 21 individually, with a fixed delay time, through cable 23. Arranged ultrasonic resonators 21 carry out transmitting and receiving processes of ultrasonic waves to and from the inside of a subject. By carrying out transmitting and receiving processes of ultrasonic waves to and from the inside of the subject with a delay time, a two-dimensional ultrasonic image can be obtained in real time.

Moreover, ultrasonic transducer 20 mechanically receives rotation movements of motor 25 through transmission axis 24. Motor 25 moves ultrasonic transducer 20 to rotate in a direction orthogonal to the aligning direction of ultrasonic resonators 21 so that ultrasonic resonators 21 thus arranged are allowed to carry out a scanning process. With this arrangement, an ultrasonic image can be obtained also in a direction orthogonal to the direction of ultrasonic resonators 21 thus arranged so that a three-dimensional ultrasonic image can be obtained.

Patent Literature 1 describes an ultrasonic transducer in which an acoustic lens or the like is attached to each ultrasonic resonator. The acoustic lens allows an acoustic matching layer and an ultrasonic beam to be focused so that its resolution can be improved. An ultrasonic transducer in which the acoustic lens is attached to its ultrasonic resonator makes it possible to exert improved sensitivity and frequency characteristic.

An electron scanning-type ultrasonic diagnostic apparatus has a structure in which a plurality of ultrasonic resonators that are arranged are classified into desired groups, and the resonators are driven, with a fixed delay time being given to each of the ultrasonic resonators, and transmitting and receiving processes of ultrasonic waves are carried between the ultrasonic resonators and the inside of a subject, and by sequentially switching the ultrasonic resonators on an electronic basis, an ultrasonic beam is variably changed so as to carry out a scanning process so that an ultrasonic image can be obtained. In Patent Literature 5, by mechanically moving these ultrasonic resonators in a direction orthogonal to an aligning direction of the ultrasonic resonators so as to carry out a scanning process by an ultrasonic beam so that a three-dimensional image is obtained. The directions of transmitting and receiving processes of the ultrasonic beam are variably changed so as to obtain a two-dimensional or three-dimensional ultrasonic image, and at present, this system has already been known as a generally-used system.

In this manner, the method for variably changing the directions of transmitting and receiving processes of an ultrasonic beam has an advantage in that a plurality of arranged ultrasonic resonators are prepared, and by switching these on an electronic basis, a high-speed operation is available, and this system is **characterized in that** an ultrasonic image can be obtained in real time.

As another method for obtaining a three-dimensional image by carrying out a scanning process, with the direction of an ultrasonic beam being variably changed, a so-called two-dimensional array-type ultrasonic transducer in which ultrasonic resonators are two-dimensionally arranged can be proposed.

Patent Literature 6 describes an ultrasonic transducer in which on ultrasonic resonators two-dimensionally arranged, a two-dimensional scanning process is carried out by an ultrasonic beam, with a variably changed electrical signal being individually applied thereto, so that a three-dimensional ultrasonic image is obtained. By carrying out the scanning process on the ultrasonic resonators two-dimensionally arranged by using an ultrasonic beam, with a variably changed electrical signal being individually applied thereto, three-dimensional image data can be obtained from the inside of a subject.

Citation List

Patent Literature

PTL 1
Japanese Patent Application Laid-Open No. 2003-47084
PTL 2
Japanese Patent Application Laid-Open No. 02-93362
PTL 3
Japanese Patent Application Laid-Open No. 2001-3776
PTL 4
Japanese Patent Application Laid-Open No. 2003-75036
PTL 5
Japanese Patent Application Laid-Open No. 2007-152101
PTL 6
US Patent No. 06238346 Specification

Summary of Invention

Technical Problem

In the electron scanning-type ultrasonic diagnostic apparatus, a plurality of ultrasonic resonators that are arranged are classified into desired groups, and the resonators are driven, with a fixed delay time being given to each of the piezoelectric elements, so that transmitting and receiving processes of ultrasonic waves are carried between the ultrasonic resonators and the inside of a subject. By giving such a delay time, an ultrasonic beam is focused or diffused so that an ultrasonic image with a wider viewing width or high resolution can be obtained.

In this case, the focus or diffusion of the ultrasonic beam in a direction orthogonal to the direction of the ultrasonic resonators thus arranged is carried out by allowing an acoustic lens to focus the beam virtually at one point so as to achieve high resolution; however, it is not possible to focus the ultrasonic beam in a depth direction of the subject relative to the electron scanning direction, with variably changing focal points of the beam.

This structure has been already known as a generally-used system. What is important to obtain an ultrasonic image with high resolution by using an ultrasonic probe is to provide a method by which the focus or diffusion of an ultrasonic beam can be controlled also in a direction orthogonal to the direction of arranged ultrasonic resonators. As one of the methods, a method has been proposed in which a plurality of ultrasonic resonators are two-dimensionally arranged to form a so-called two-dimensional array so that the ultrasonic beam is controlled on an electronic basis. However, it is very difficult to provide the corresponding electronic circuit, controlling process and ultrasonic transducer, and at present, this system has not yet been easily achieved.

Patent Literature 1 has proposed a structure in which void portions are prepared at two ends of an acoustic lens having a curvature, with a liquid being allowed to flow through the void portions, so that, by adjusting the flow rate, the curvature of the acoustic lens is variably changed to consequently variably change the position of the focal point of ultrasonic waves. However, this system requires a device for adjusting the flow rate of the liquid, and also causes a problem in that the variably changing process is not carried out at high speeds.

Moreover, Patent Literature 2 has proposed a structure in which a piezoelectric bimorph is formed on the backface of the ultrasonic resonator so that the ultrasonic resonator or the like is deformed so as to variably change the position of the focal point of an ultrasonic beam. In this structure, however, it is necessary to deform the entire ultrasonic resonator, with the result that the ultrasonic resonator is limited to a member with flexibility, and other important characteristics, such as sensitivity and frequency characteristics, are also limited.

Moreover, Patent Literature 3 has proposed a structure in which a shape memory alloy is formed on the backface of an ultrasonic resonator so that the shape memory alloy is deformed by a temperature change, with the result that the ultrasonic resonator or the like is deformed to variably change the position of the focal point of an ultrasonic beam. However, it is difficult to carry out the variably changing process of the temperature at high speeds, and in the same manner as in the above-mentioned structure, it is necessary to deform the entire ultrasonic resonator, with the result that the ultrasonic resonator is limited to a member with flexibility, and other important characteristics, such as sensitivity and frequency characteristics, are also limited.

Furthermore, Patent Literature 4 has proposed a structure in which an acoustic-velocity variably changing layer is formed on the front face of an ultrasonic resonator so that by variably changing the acoustic velocity, a change in propagating time of ultrasonic waves is prepared so as to variably change the focal position of an ultrasonic beam. However, although the focusing position of an ultrasonic beam can be variably changed, the acoustic-velocity variably changing layer is formed between the ultrasonic resonator and a subject, with the result that since the acoustic matching is influenced, problems with other important characteristics, such as sensitivity and frequency characteristics, are raised.

There have been strong demands for high resolution for ultrasonic images, and so as to meet these demands, further developments of technique have been required.

Moreover, the electron scanning-type ultrasonic diagnostic apparatus, as shown in Patent Literature 5, carries out a scanning process by an ultrasonic beam by mechanically rotating an ultrasonic transducer, or carrying out parallel or rocking movements thereon, by the use of a motor and a driving system that transmits the driving force. Since this structure uses the motor and driving system, it is impossible to provide a light-weight system with a small size, and another problem is that it is difficult to operate the system. Moreover, since there are changes with age such as wearing of mechanisms or the like, and since the service life of electric terminal cables or the like drawn from each ultrasonic resonator by vibration is not long, problems are raised in reliability.

Furthermore, in the method for two-dimensionally scanning the ultrasonic beam with variably changing an electrical signal for each of ultrasonic resonators two-dimensionally arranged, as shown in Patent Literature 6, the number of ultrasonic resonators becomes very large, and the number of cables to be connected to electric terminals drawn from the respective ultrasonic resonators also becomes very large. This structure is too complicated to construct an ultrasonic transducer to cause difficulty in easily providing the system. Moreover, the number of control circuits becomes greater in response to an increase of ultrasonic resonators, resulting in a problem of a bulky circuit scale.

It is therefore an object of the present invention to provide an ultrasonic transducer, an ultrasonic probe and an ultrasonic diagnostic apparatus that allow an ultrasonic beam in a depth direction for diagnosis to be linearly focused over a wide range, and provide a diagnostic image with high resolution. Another object of the present invention is to provide an ultrasonic transducer and an ultrasonic probe that can variably change the direction of an ultrasonic beam by changing the shape of a shape-changing layer, and can carry out a three-dimensional scanning process by an ultrasonic beam, by using a simple structure having a small size and light weight.

Solution to Problem

An ultrasonic transducer in accordance with the present invention is provided with: an ultrasonic resonator which transmits and receives ultrasonic waves, a shape-changing layer which is disposed on a subject side of the ultrasonic resonator and a shape of which changes upon receipt of an electrical signal, and a control section which controls the electrical signal to apply to the shape-changing layer, and in this structure, the control section changes the shape of the shape-changing layer by controlling the electrical signal to apply to the shape-changing layer so that the direction of ultrasonic waves can be variably changed.

The ultrasonic transducer of the present invention is provided with: an ultrasonic resonator array in which ultrasonic resonators for transmitting and receiving ultrasonic waves are arranged one-dimensionally, a shape-changing layer which is disposed on a subject side of the ultrasonic resonator and a shape of which changes upon receipt of an electrical signal, and a control section that controls the electrical signal to apply to the shape-changing layer, and in this structure, the control section controls the electrical signal to apply to the shape-changing layer in cooperation with a focus of ultrasonic waves in an aligning direction of the ultrasonic resonators, with the shape of the shape-changing layer being changed in a direction orthogonal to the ultrasonic resonator array, so that the ultrasonic waves are variably changed in focus or diffusion.

The ultrasonic transducer of the present invention is provided with: an ultrasonic resonator which transmits and receives ultrasonic waves, a shape-changing layer which is disposed on a side opposite to the subject side of the ultrasonic resonator and a shape of which changes upon receipt of an electrical signal, and, in this structure, by controlling the electrical signal, the shape of the shape-changing layer is changed so that the direction of an ultrasonic beam is variably changed.

An ultrasonic probe in accordance with the present invention is provided with the ultrasonic transducer, and a shape-changing layer, installed on one portion of the ultrasonic transducer and a shape of which changes by an electrical signal, and, in this structure, by controlling the electrical signal, the shape of the shape-changing layer is changed to consequently variably change the direction of the ultrasonic transducer so that the direction of an ultrasonic beam is variably changed.

An ultrasonic diagnostic apparatus in accordance with the present invention, which is an ultrasonic diagnostic apparatus in which by applying a pulse voltage to an ultrasonic resonator, ultrasonic waves are transmitted, and received ultrasonic waves are focused or diffused, and converted to an electrical signal so that an ultrasonic diagnosis is carried out, is provided with an ultrasonic probe including: an ultrasonic resonator array in which ultrasonic resonators which transmit and receive ultrasonic waves are arranged; a shape-changing layer a shape of which changes upon receipt of an electrical signal; and a control section which controls the electrical signal to apply to the shape-changing layer, and, in this structure, the control section comprises an ultrasonic probe which controls the electrical signal to apply to the shape-changing layer in cooperation with a focus of ultrasonic waves in an aligning direction of the ultrasonic resonators, with the shape of the shape-changing layer changing in a direction orthogonal to the ultrasonic resonator array, so that the ultrasonic waves are variably changed in focus or diffusion.

Advantageous Effects of Invention

In accordance with the present invention, by changing the shape of the shape-changing layer by applying an electrical signal thereto, an ultrasonic beam in a depth direction for diagnosis can be linearly focused over a wide range so that a diagnostic image with high resolution can be obtained. Moreover, by variably changing the shape of the shape-changing layer into a desired shape, the focus or diffusion of the ultrasonic beam is freely and variably controlled so that a diagnostic image with high resolution can be obtained.

In accordance with the present invention, by changing the shape of the shape-changing layer by applying an electrical signal thereto, a three-dimensional scanning process is carried out by an ultrasonic beam, by using a simple structure with a small size and light weight so that an ultrasonic image with high resolution can be obtained.

Brief Description of Drawings

FIG.1 is a schematic cross-sectional view that shows a conventional ultrasonic transducer;

FIG.2 is a schematic view that shows a structure of the conventional ultrasonic transducer;

FIG.3 is a cross-sectional view that shows a schematic structure of an ultrasonic transducer in accordance with embodiment 1 of the present invention;

FIG.4 is a perspective view that shows a schematic structure of an ultrasonic transducer in accordance with embodiment 2 of the present invention;

FIG.5 is a cross-sectional view that shows a schematic structure of an ultrasonic transducer in accordance with embodiment 3 of the present invention;

FIG.6 is a view that shows a schematic structure of an ultrasonic transducer in accordance with embodiment 4 of the present invention;

FIG.7 is a perspective view that shows a schematic structure of an ultrasonic transducer in accordance with embodiment 5 of the present invention;

FIG.8 is a cross-sectional view that shows a schematic structure of an ultrasonic probe in accordance with embodiment 6 of the present invention;

FIG.9 is a cross-sectional view that shows a schematic structure of an ultrasonic transducer in accordance with embodiment 7 of the present invention;

FIG.10 is a perspective view that shows a schematic structure of an ultrasonic transducer in accordance with embodiment 8 of the present invention;

FIG.11 is a cross-sectional view that shows a schematic structure of an ultrasonic transducer in accordance with embodiment 9 of the present invention;

FIG.12 is a view that shows a schematic structure of an ultrasonic transducer in accordance with embodiment 10 of the present invention;

FIG.13 is a cross-sectional view that shows a schematic structure of an ultrasonic probe in accordance with embodiment 11 of the present invention;

FIG.14 is a cross-sectional view that shows a schematic structure of an ultrasonic transducer in accordance with embodiment 12 of the present invention;

FIG.15 is a perspective view that shows a schematic structure of the ultrasonic transducer in accordance with embodiment 12 of the present invention;

FIG.16 is a cross-sectional view that shows a schematic structure of an ultrasonic transducer in accordance with embodiment 13 of the present invention; and

FIG.17 is a cross-sectional view that shows a schematic structure of an ultrasonic probe in accordance with embodiment 14 of the present invention.

Description of Embodiments

Referring to the drawings, the following description will discuss embodiments of the present invention in detail.

(Embodiment 1)

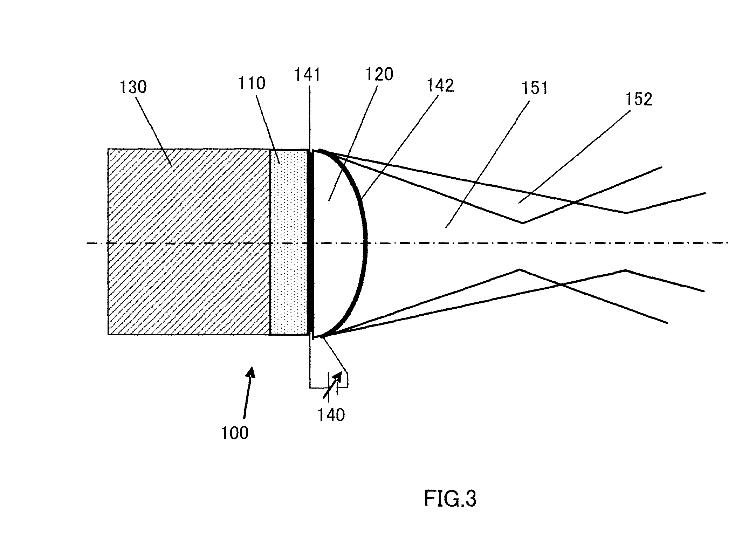

FIG.3 is a cross-sectional view that shows a schematic structure of an ultrasonic transducer in accordance with an embodiment 1 of the present invention.

As shown in FIG.3, an ultrasonic transducer 100 is provided with an ultrasonic resonator 110, a shape-changing layer 120, a backing material 130, electrodes (first electrode) 141 and (second electrode) 142 that are disposed on the two surfaces of shape-changing layer 120, and a variable power supply 140 that applies an electrical signal between electrodes 141 and 142.

Moreover, if necessary, one or more acoustic matching layers (not shown) may be installed between ultrasonic resonator 110 and shape-changing layer 120. These components other than shape-changing layer 120 respectively have the same functions as those explained in the prior art by reference to FIG.1.

Ultrasonic resonator 110 is made of a material such as a piezoelectric ceramic material of PZT based material, a piezoelectric single crystal such as PZN-PT or PMN-PT-based crystal, or a composite piezoelectric material that is a composite material between the above-mentioned material and a polymer. Moreover, in addition to these, a static capacity-type material, formed by applying a micromachining technique to a silicon semiconductor, may be used; thus, ultrasonic resonator 110 mentioned here is not intended to be limited by these, as long as it serves as an ultrasonic sensor that can transmit and receive ultrasonic waves.

A grounding electrode, not shown, is formed on the front face of ultrasonic resonator 110 in the thickness direction, and a signal electrode is formed on the backface thereof respectively. The two electrodes are respectively formed on the front face and backface of ultrasonic resonator 110, by using vapor deposition of gold or silver, a sputtering process, or a silver enameling process.

Shape-changing layer 120, which is placed on the front face in the thickness direction corresponding to the subject side (right side in FIG.3) relative to ultrasonic resonator 110, has functions for focusing or diffusing an ultrasonic beam.

In the case when an acoustic velocity has a value that is different from the acoustic velocity (1540 m/s.) of a subject, for example, an organism, shape-changing layer 120 allows an ultrasonic beam to be focused, or to be also diffused, with a focal point at a desired distance. These functions are derived from the fact that, by making a difference in velocities between the acoustic velocity of shape-changing layer 120 and the acoustic velocity of the subject, ultrasonic waves are refracted on the interface thereof so that a lens function is obtained by utilizing this refraction. For example, in the case when, with the acoustic velocity of shape-changing layer 120 being slower than the acoustic velocity of the organism (1540 m/s.), the ultrasonic beams 151 and 152 are focused with focal points being set at desired distances, the shape of the surface on the subject side of shape-changing layer 120 has a convex surface shape; in contrast, with the acoustic velocity of shape-changing layer 120 being faster, the shape of the surface on the subject side thereof has a concave surface shape in a reversed manner. Thus, shape-changing layer 120 exerts functions of an acoustic lens.

Although the acoustic impedance (density x acoustic velocity) of shape-changing layer 120 is desirably set to a value close to the value (about 1.6 MRayls) of the subject (organism), the acoustic impedance may be set to a value between that of ultrasonic resonator 110 and that of the subject.

Conventionally, the acoustic lens generally has a structure in which a silicone rubber material is used, and the acoustic velocity of this material is about 1000 m/s., with the result that, in the case when the ultrasonic beams 151 and 152 are focused, with their focal points being set to desired distances, the surface shape forms a convex surface. This shape is allowed to have a fixed curvature, and because of this shape, a focus is made by either the ultrasonic beam 151 or 152 fixed with a focal point being set to a certain distance.

In the present embodiment, shape-changing layer 120 is formed by using a material that can be variably changed in its shape on the subject side by applying an electrical signal. Electrodes 141 and 142 are formed on the two surfaces of shape-changing layer 120, and an electrical signal (in this case, voltage) is applied between electrodes 141 and 142. Variable power supply 140 adjusts the voltage to apply between electrodes 141 and 142. Thus, the shape of shape-changing layer 120 is changed so that the focus or diffusion of the ultrasonic beams 151 and 152 is controlled.

As the material of shape-changing layer 120, materials, such as an ionic polymer metal composite, a dielectric polymer, and a conductive polymer, whose polymer is deformed upon application of an electrical signal to its polymer are used.

The ionic polymer metal composite forms a polymer actuator made of an ion exchange resin with electrodes formed on its two surfaces, and ions inside the ion exchange resin are moved by an applied voltage so that the side on which the ions have been moved is swelled to make the polymer deformed. As the material of this polymer actuator, those materials, prepared by introducing a functional group, such as a sulfonic acid group and a carboxyl group, into a material such as polyethylene, polystyrene, and a fluorine resin, and polymeric materials in which, as disclosed by Patent Literature 5, a non-conductive polymer, such as polyvinyl chloride (PVC), polymethyl methacrylate (PMMA) and polyurethane, and an ionic material are contained, may be used.

The dielectric polymer is designed so that by applying a voltage between electrodes formed on the two surfaces of a polymer member, the polymer is compressed in its thickness direction by an electrostatic attracting force exerted between the electrodes, with the result that the polymer is expanded in its plane direction, and deformed. As this dielectric polymer, materials such as silicone rubber, polyurethane, and acrylic elastomer, may be used.

Moreover, in the case of the conductive polymer, electric terminals are drawn from a conductive polymer, and upon application of a voltage across the terminals, the conductive polymer between the electric terminals is contracted, while, upon turning the applied voltage off, the polymer is returned to its original state. As the dielectric conductive polymer, a polypyrrole resin or the like may be used.

In addition to the above-mentioned materials, any material may be used as long as its polymer material is deformed by an electrical signal, and the present invention is not intended to be limited by the above-mentioned materials.

In this case, the electricity of the electrical signal is mainly voltage-adjusted; however, this may be electric-current-adjusted. Moreover, the signal of the above-mentioned electrical signal is expressed as a signal by including arrangements in which its voltage is made variable, or applied timing or the like is made variable.

Backing material 130, which is designed so as to mechanically hold ultrasonic resonator 110 from the backface thereof, is disposed on the backface (left side in FIG.3) in the thickness direction corresponding to the opposite side to shape-changing layer 120 relative to ultrasonic resonator 110, so as to exert a function for attenuating an unnecessary ultrasonic signal, if necessary.

Next, in the case of using silicone rubber corresponding to the dielectric polymer as shape-changing layer 120, the focal distance f of an ultrasonic beam and the curvature radius R of the silicone rubber are indicated by the following equation 1:

$$f = R/((C2/C1) - 1)) \qquad \dots \text{(Equation 1)}$$

In this case, C1 represents the acoustic velocity of silicone rubber, and C2 represents the acoustic velocity of a subject (organism). The acoustic velocity C1 of silicone rubber is about 1000 m/s., and the acoustic velocity of the subject is 1540 m/s. Supposing that the width of shape-changing layer 120 of silicone rubber (in the vertical direction in FIG.3) is 5 mm, and that the largest thickness of shape-changing layer 120 (in the proceeding direction of an ultrasonic beam in FIG.3) is H, in order to set a focal distance f to which the ultrasonic beam is focused to 50 mm, the curvature radius R of the change layer is about 27 mm, and the thickness H becomes 0.116 mm, from the above-mentioned formula.

In this state, variable power supply 140 applies a voltage across electrodes 141 and 142 of shape-changing layer 120. In response to the electrical signal of the applied voltage, shape-changing layer 120 changes its shape so as to variably change the curvature radius.

For example, in order to set the focal distance f for focus of an ultrasonic beam to 20 mm, the curvature radius R of the shape-changing layer becomes about 11 mm and the thickness H becomes 0.288 mm from the above-mentioned equation 1. This change in the curvature radius is obtained by adjusting the applied voltage by variable power supply 140.

The dielectric polymer is designed such that, by applying a voltage across electrodes 141 and 142, the polymer is compressed in the thickness direction by an electrostatic attracting force, and deformed. For example, in a state where, with the curvature radius being set to about 11 mm and the thickness H being set to 0.288 mm, no voltage is applied across electrodes 141 and 142, and from this state, a voltage is applied across the electrodes, and the shape is changed by an electrostatic attracting force so that the thickness is made thinner. In this case, the dielectric polymer can be set to about 27 mm in its curvature radius. In the dielectric polymer, the applied voltage and the shape change are directly proportional to each other so that it has a characteristic in which as the applied voltage becomes higher, the degree of a shape change becomes greater.

In this manner, by making the voltage to apply to shape-changing layer 120 variable, the focal distance for focus of the ultrasonic beam is desirably made variable, and since shape-changing layer 120 itself can be deformed, the variably changing process can be carried out at high speeds.

The above description has been given by exemplifying the structure in which silicone rubber is used as shape-changing layer 120.

Materials other than silicone rubber may be used. For example, polymeric materials, such as an ionic polymer metal composite, a dielectric polymer and a conductive polymer, may be used, and in this case, the curvature radius is designed to be changed in association with the value of the acoustic velocity of the material of shape-changing layer 120.

Additionally, in the case when polyethylene, polystyrene, fluorine resin, or the like is used as the ionic polymer metal composite for use as a polymer actuator, since its acoustic velocity becomes about 2000 m/s faster than the acoustic velocity 1540 m/s of a subject (organism), the surface is formed into a concave surface shape so as to allow an ultrasonic beam to be focused.

As described above in detail, ultrasonic transducer 100 of the present embodiment is provided with ultrasonic resonator 110, shape-changing layer 120, formed on the subject side of the ultrasonic resonator and a shape of which changes by an electrical signal, electrodes 141 and 142 that are disposed on the two surfaces of shape-changing layer 120, and variable power supply 140 that applies an electrical signal between electrodes 141 and 142, and variable power supply 140 changes the shape of shape-changing layer 120 by controlling the electrical signal to be applied between electrodes 141 and 142 so that an ultrasonic beam can be variably focused or diffused. Since the shape of shape-changing layer 120 is changed, and since the curved surface of the surface of shape-changing layer on the subject side is consequently changed, the focal distance for focus of the ultrasonic beam that is transferred through shape-changing layer 120 can be variably changed desirably.

Moreover, since a diffusing process is also available, the ultrasonic beam can be focused linearly over a wide range in the depth direction of an ultrasonic image so that an ultrasonic image with high resolution can be obtained.

Furthermore, by variably changing the voltage to apply to shape-changing layer 120 at high speeds, the ultrasonic image is allowed to have high resolution in real time.

The deformation is made only in shape-changing layer 120, with ultrasonic resonator 110, backing material 130 or an acoustic matching layer to be installed, if necessary, being secured without being changed. For this reason, with respect to ultrasonic resonator 110, backing material 130 or the acoustic matching layer to be installed, if necessary, since no limitations are required for materials for use in deformation, it is possible to provide a transducer with high performances.

(Embodiment 2)

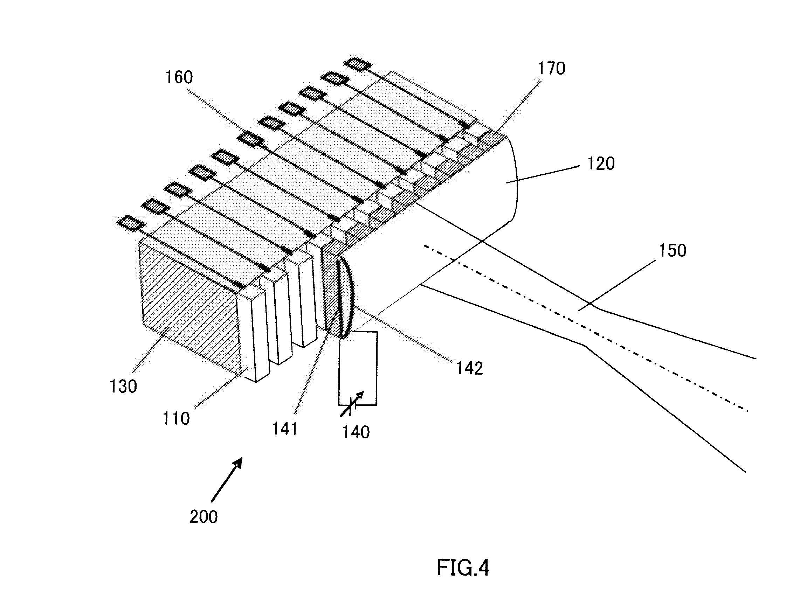

FIG.4 is a perspective view that shows a schematic structure of an ultrasonic transducer in accordance with an embodiment 2 of the present invention. The same functional portions as those of FIG.3 are indicated by the same reference numerals, and overlapping explanations will not be repeated.

As shown in FIG.4, ultrasonic transducer 200 is provided with a plurality of ultrasonic resonators 110 that are arranged, acoustic matching layer 170 that is disposed on the front face in the thickness direction corresponding to the subject side (right side in FIG.4) in association with ultrasonic resonators 110, and backing material 130 that is disposed on the backface (left side in FIG.4) in the thickness direction corresponding to the opposite side to acoustic matching layer 170 relative to ultrasonic resonator 110, if necessary. Moreover, ultrasonic transducer 200 is also provided with shape-changing layer 120 disposed on acoustic matching layer 170, electrodes 141 and 142 that are disposed on the two surfaces of shape-changing layer 120 and variable power supply 140 that applies an electrical signal between electrodes 141 and 142. This structure is referred to as a so-called electronic scanning type ultrasonic transducer.

Ultrasonic resonators 110 are made of a material such as a piezoelectric ceramic material of PZT based material, a piezoelectric material such as PZN-PT or PMN-PT-based material, or a composite piezoelectric material that is a composite material between the above-mentioned piezoelectric material and a polymer material. Moreover, in addition to these, a static capacity-type material, formed by applying a micromachining technique to a silicon semiconductor, may be used; thus, ultrasonic resonators 110 mentioned here are not intended to be limited by these materials, as long as they serve as ultrasonic sensors that can transmit and receive ultrasonic waves.

A grounding electrode (not shown in FIGS) is formed on the front face of each of ultrasonic resonators 110, and a signal electrode (not shown in FIGS) is formed on the backface of each of ultrasonic resonators 110. The grounding electrode and signal electrode are respectively formed, by plating gold or silver, vapor deposition, a sputtering process, or a silver enameling process. Into the signal electrode, signal electric terminal 160, formed by depositing a metal film such as copper on an insulating film made from a polymeric material such as polyimide, is inserted. The signal electrode is connected from the grounding electrode formed on each ultrasonic resonator 110 to a grounding electric terminal (not shown), in the same manner as in signal electric terminal 160.

The following description will discuss operations of ultrasonic transducer 200 having the above-mentioned structure.

The signal electrode formed on each ultrasonic resonator 110 is electrically connected to one end of each of cables, not shown, through signal electric terminal 160, or through the grounding electrode of each ultrasonic resonator 110, and the other end of each of the cables is connected to a main unit section of an ultrasonic diagnostic apparatus, not shown, through a connector or the like. With this arrangement, a pulse voltage, prepared in the main unit section of the ultrasonic diagnostic apparatus, is applied to each ultrasonic resonator 110 so that ultrasonic waves are transmitted, and echoes of the received ultrasonic waves are converted into electrical signals and transmitted to the main unit section of the ultrasonic diagnostic apparatus. As this system that is mainly used, systems have been generally adopted in which a phase-controlling process is carried out by providing a time delay upon transmitting and receiving signals to each of arranged ultrasonic resonators 110 so that the ultrasonic beam is focused to a desired position so as to provide high resolution, or in which the ultrasonic beam is deflected so as to carry out a scanning process in a delta form.

Shape-changing layer 120 has such a function as to focus an ultrasonic beam in a direction orthogonal to plural ultrasonic resonators 110 that are arranged so that the resolution of an ultrasonic image is improved. Shape-changing layer 120 is designed such that, when the acoustic velocity of shape-changing layer 120 is a value that is different from the acoustic velocity (1540 m/s.) of a subject, for example, an organism, it allows ultrasonic beam 150 to be focused, or to be also diffused, with a focal point being set at a desired distance. These functions are derived from the fact that, by making a difference in velocities between the acoustic velocity of shape-changing layer 120 and the acoustic velocity of the subject, ultrasonic waves are refracted on the interface thereof so that a lens function is obtained by utilizing this refraction. For example, in the case when, with acoustic velocity of shape-changing layer 120 being slower than the acoustic velocity of the organism (1540 m/s.), ultrasonic beams 150 is focused with its focal point being set at a desired distance, the shape of the surface on the subject side of shape-changing layer 120 has a convex surface shape; in contrast, with the acoustic velocity of shape-changing layer 120 being faster, the shape of the surface on the subject side thereof has a concave surface shape in a reversed manner.

Conventionally, the acoustic lens in which a silicone rubber material is used generally exerts this function. The acoustic velocity of this material is about 1000 m/s.; therefore, in the case when the ultrasonic beam is focused, with its focal point being set to a desired distance, its surface shape forms a convex surface. The shape of the acoustic lens is allowed to have a fixed curvature, and because of this shape, ultrasonic beam 150 that is fixed with a focal point being set to a certain distance is desirably focused.

In the present embodiment, shape-changing layer 120 is formed by using a material that can be variably changed in its shape on the subject side by applying an electrical signal thereto. Electrodes 141 and 142 are formed on the two surfaces of shape-changing layer 120, and an electrical signal (in this case, voltage) is applied between electrodes 141 and 142. Variable power supply 140 adjusts the voltage to apply between electrodes 141 and 142. In shape-changing layer 120, the degree of its change and the voltage are virtually directly proportional to each other so that shape-changing layer 120 has a characteristic in which as the voltage becomes higher, the degree of its change becomes greater. Thus, by changing the shape of shape-changing layer 120, the focus of ultrasonic beam 150 can be controlled, or the diffusion thereof can be controlled.

As materials for shape-changing layer 120, materials, such as an ionic polymer metal composite, a dielectric polymer, and a conductive polymer, whose polymer is deformed upon application of an electrical signal to its polymer are used as in embodiment 1.

As shown in FIG.4, shape-changing layer 120 has its curved surface shape aligned in a direction (vertical direction in the FIG.) orthogonal to the aligning direction (a direction from the lower left side to the upper right side of the FIG.) of ultrasonic resonators 110 (in other words, the curved surface is formed in such a manner as to draw a curved line on a plane including the axis (vertical direction in the FIG.) of a direction orthogonal to the aligning direction (a direction connecting the lower left side to the upper right side in the FIG.) of ultrasonic resonators 110 and the axis of transmitting and receiving directions of the ultrasonic waves), and the curvature radius is variably changed in response to a voltage to be applied between electrodes 141 and 142 of shape-changing layer 120 so that ultrasonic beam 150, focused to a desired position relative to the curved surface, can be adjusted. On the other hand, the aligning direction of ultrasonic resonators 110 can be adjusted by carrying out a phase-controlling process, by providing a time delay upon transmitting and receiving signals to each of arranged ultrasonic resonators 110, so that the ultrasonic beam is adjusted to be focused to a desired position. In the case of transmission, the aligning direction of the array of ultrasonic resonators 110 can be switched to a concerned region as a desired position by the ultrasonic diagnostic apparatus.

In this manner, an application method for improving the probability of appropriate diagnosis, with a diagnosis image of a concerned region being obtained, is proposed.

In order to provide a diagnosis image with higher resolution, the present embodiment is designed to change the shape of shape-changing layer 120 in a direction orthogonal to the aligning direction of the array of ultrasonic resonators 110. Moreover, the aligning direction of the array of ultrasonic resonators 110 is changed in association with the switching of the focus beam of ultrasonic waves upon transmission. With these arrangements, the ultrasonic beam is focused to the concerned region. Since the ultrasonic beam can be focused two-dimensionally, it is possible to obtain an ultrasonic image with higher resolution.

Moreover, variable power supply 140 to apply an electrical signal between electrodes 141 and 142 may be connected in a manner so as to be operated in cooperation with the switching process of a position at which the ultrasonic beam is conversed upon transmission by the ultrasonic diagnostic apparatus.

In other words, the switching process of the position at which ultrasonic beam 150 is focused upon transmission by the ultrasonic diagnostic apparatus is a normally executed operation so as to provide high resolution to the concerned region and consequently to improve the probability of appropriate diagnosis. The aligning direction of ultrasonic resonators 110 is phase-controlled by providing a delay time thereto upon transmitting and receiving a signal to and from each of ultrasonic resonators 110 so that the ultrasonic beam can be focused at a desired position. In cooperation with this, with respect to the direction in which ultrasonic beam 150 is focused by shape-changing layer 120 placed in a direction orthogonal to the aligning direction of ultrasonic resonators 110 as well, focuses are made at one portion or a plurality portions within the concerned region to be diagnosed. Thus, it becomes possible to obtain an ultrasonic image with by far higher resolution.

Although the explanation has been given of a structure in which the ultrasonic beam is transmitted in the aligning direction of the array of ultrasonic resonators 110, the present embodiment is not intended to be limited by this structure, and the same effects can be obtained even by using a structure in which, by changing the shape of shape-changing layer 120, the focus of ultrasonic beam 150 is variably changed, in cooperation with the receiving side, or both of the transmitting and receiving sides.

The following description will discuss a structure in which, for example, silicone rubber is used as shape-changing layer 120.

The focal distance f of ultrasonic beam 150 and the curvature radius R of the silicone rubber are indicated by the aforementioned equation 1.

In the above-mentioned equation 1, C1 represents the acoustic velocity of silicone rubber, and C2 represents the acoustic velocity of a subject (organism). The acoustic velocity C1 of silicone rubber is about 1000 m/s., and the acoustic velocity of the subject is 1540 m/s. Supposing that the width of shape-changing layer 120 of silicone rubber (in a direction orthogonal to the aligning direction of ultrasonic resonators 110 in FIG.4) is 5 mm, and that the thickness of shape-changing layer 120 (in the proceeding direction of an ultrasonic beam in FIG.4) is H, in order to set a focal distance f to which the ultrasonic beam is focused to 50 mm, the curvature radius R of the change layer is about 27 mm, and the thickness H becomes 0.116 mm, from the above-mentioned equation 1.

In this state, variable power supply 140 applies a voltage across electrodes 141 and 142 of shape-changing layer 120. In response to the electrical signal of the applied voltage, shape-changing layer 120 changes its shape so as to variably change the curvature radius.

For example, in order to set the focal distance f for focus of an ultrasonic beam to 20 mm, the curvature radius R of the shape-changing layer becomes about 11 mm and the thickness H becomes 0.288 mm from the above-mentioned equation 1. This change in the curvature radius is obtained by adjusting the applied voltage by variable power supply 140.

The dielectric polymer is designed such that, by applying a voltage across electrodes 141 and 142, the polymer is compressed in the thickness direction by an electrostatic attracting force, and deformed. For example, in a state where, with the curvature radius being set to about 11 mm and the thickness H being set to 0.288 mm, no voltage is applied across electrodes 141 and 142, and from this state, a voltage is applied across the electrodes, and the shape is changed by an electrostatic attracting force so that the thickness is made thinner. In this case, the dielectric polymer can be set to about 27 mm in its curvature radius. In the dielectric polymer, the applied voltage and the shape change are directly proportional to each other so that it has a characteristic in which as the applied voltage becomes higher, the degree of a shape change becomes greater.

In this manner, by making the voltage to apply to shape-changing layer 120 variable, the focal distance for focus of the ultrasonic beam is desirably made variable, and since shape-changing layer 120 itself can also be deformed, the variably changing process can be carried out at high speeds.

The above description has been given by exemplifying the structure in which silicone rubber is used as shape-changing layer 120.

Materials other than silicone rubber may be used. For example, polymeric materials, such as an ionic polymer metal composite, a dielectric polymer and a conductive polymer, may be used, and in this case, the curvature radius is designed to be changed in association with the value of the acoustic velocity of the material of shape-changing layer 120.

Additionally, in the case when polyethylene, polystyrene, fluorine resin, or the like is used as the ionic polymer metal composite for use as a polymer actuator, since its acoustic velocity becomes about 2000 m/s faster than the acoustic velocity 1540 m/s of a subject (organism), the surface is formed into a concave surface shape so as to allow ultrasonic beam 150 to be focused.

In accordance with the present embodiment, in the same manner as in embodiment 1, by variably changing the voltage to apply to shape-changing layer 120, the shape of shape-changing layer 120 is changed, with the result that the curved surface of the surface on the subject side of shape-changing layer 120 is consequently changed. Thus, ultrasonic beam 150 that is transferred through shape-changing layer 120 can be variably changed desirably. For this reason, the ultrasonic beam can be focused linearly over a wide range in the depth direction of an ultrasonic image so that an ultrasonic image with high resolution can be obtained.

Furthermore, by variably changing the voltage to apply to shape-changing layer 120 at high speeds, the ultrasonic image is allowed to have high resolution in real time.

Moreover, the deformation is made only in shape-changing layer 120, with ultrasonic resonator 110, backing material 130 and an acoustic matching layer 170 to be installed, if necessary, being secured without being changed. For this reason, with respect to resonator 110, backing material 130 or acoustic matching layer 170 to be installed, if necessary, since no limitations are required for materials for use in deformation, it is possible to provide a transducer with high performances.

Furthermore, upon obtaining a cross-sectional image in ultrasonic diagnostic apparatus, by controlling so as to variably change a plurality of focal points at which the ultrasonic beam is focused, with the voltage to apply to shape-changing layer 120 being variably changed a plurality of times, it becomes possible to obtain an ultrasonic image with by far higher resolution.

In the present embodiment, as one method for providing a diagnosis image with high resolution, for example, at a desired depth to be diagnosed by using the ultrasonic diagnostic apparatus, a switching process for switching the depth at which the transmitted ultrasonic beam is focused, by carrying out an electron-scanning process in the aligned direction of ultrasonic resonators 110. The depth for focus of the ultrasonic beam by the acoustic lens that is orthogonal to the aligned direction of ultrasonic resonators 110 has been fixed in conventional systems, and has not been variably changed. In contrast, in the present embodiment, since the depth for focus of the ultrasonic beam can be variably changed desirably, ultrasonic beam 150 can be focused or diffused also in the direction orthogonal to the aligned direction of ultrasonic resonators 110, in cooperation with the depth at which the ultrasonic beam 150 transmitted from the ultrasonic diagnostic apparatus is focused.

Additionally, in the present embodiment, the explanation has been given by exemplifying a structure in which shape-changing layer 120 is formed in an arrangement in which ultrasonic resonators 110 are one-dimensionally disposed; however, the present embodiment may be applicable to a structure in which shape-changing layer 120 is formed in an arrangement in which ultrasonic resonators 110 are two-dimensionally disposed, in the same manner. For example, in the case when, as the arrangement in which ultrasonic resonators 110 are two-dimensionally disposed, an arrangement in which each of ultrasonic resonators 110, shown in FIG.4, is divided into plurality of pieces in the vertical direction in the FIG. is used, by forming the curved surface in a manner so as to draw a curved line on a plane including not only the vertical direction of FIG.4 in the same manner as described above, but also a direction that connects the lower left side to the upper right side of the FIG. that is orthogonal to this direction, and the axis of transmitting and receiving directions of ultrasonic waves, it becomes possible to obtain the same effects.

(Embodiment 3)

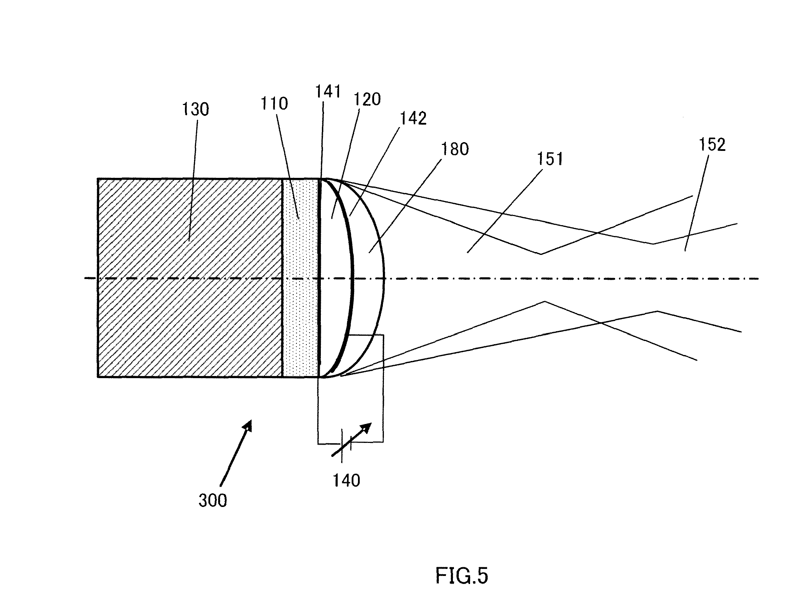

FIG.5 is a cross-sectional view that shows a schematic structure of an ultrasonic transducer in accordance with embodiment 3 of the present invention. The same functional portions as those of FIG.3 are indicated by the same reference numerals, and overlapping explanations will not be repeated.

As shown in FIG.5, ultrasonic transducer 300 is provided with an ultrasonic resonator 110, shape-changing layer 120, backing material 130, electrodes 141 and 142 that are formed on the two surfaces of shape-changing layer 120, variable power supply 140 that applies an electrical signal between electrodes 141 and 142, and an acoustic lens 180 that is disposed on the front face of shape-changing layer 120.

Moreover, one or more acoustic matching layers (not shown) may be formed between ultrasonic resonator 110 and shape-changing layer 120, if necessary. These components other than shape-changing layer 120 respectively have the same functions as those explained in the prior art by reference to FIG.1.

Ultrasonic resonator 110 is made of a material such as a piezoelectric ceramic material of PZT based material, a piezoelectric single crystal such as PZN-PT or PMN-PT-based crystal, or a composite piezoelectric material that is a composite material between the above-mentioned material and a polymer. Moreover, in addition to these, a static capacity-type material, formed by applying a micromachining technique to a silicon semiconductor, may be used; thus, ultrasonic resonator 110 mentioned here is not intended to be limited by these, as long as it serves as an ultrasonic sensor that can transmit and receive ultrasonic waves.

A grounding electrode, not shown, is formed on the front face of ultrasonic resonator 110 in the thickness direction, and a signal electrode is formed on the backface thereof respectively. The two electrodes are respectively formed on the front face and backface of ultrasonic resonator 110, by using vapor deposition of gold or silver, a sputtering process, or a silver enameling process.

Shape-changing layer 120, which is allowed to have an assisting function for allowing ultrasonic beams 151 and 152 to be focused with focal points thereof being set at desired distances, and in this structure, the function for setting the focal points of ultrasonic beams 151 and 152 to the desired distances to be conversed thereto is taken by acoustic lens 180.

That is, acoustic lens 180 is made by using, for example, a material, such as silicone rubber, so as to have a fixed shape. In this case, in response to the curvature of the shape of acoustic lens 180, ultrasonic beam 151 or 152 is focused at a certain distance.

In the present embodiment, shape-changing layer 120 is made by using a material that allows the shape on the subject side thereof to be variably changed desirably by applying an electrical signal thereto. Electrodes 141 and 142 are formed on the two surfaces of shape-changing layer 120, with an electrical signal (voltage in this case) being applied between electrodes 141 and 142. Variable power supply 140 adjusts the voltage to apply between electrodes 141 and 142. Thus, shape-changing layer 120 is deformed. By allowing acoustic lens 180 formed on the front face to be also deformed following this deformation, the curvature of acoustic lens 180 is also variably changed. In response to the deformation of the curvature of acoustic lens 180, focal distances for focus of ultrasonic beams 151 and 152 can be also variably changed.

As materials for shape-changing layer 120, materials, such as an ionic polymer metal composite, a dielectric polymer, and a conductive polymer, whose polymer is deformed upon application of an electrical signal to its polymer are used in the same manner as in embodiment 1.

Additionally, shape-changing layer 120 to be used here has a function for allowing acoustic lens 180 to be deformed to variably change the curvature. For this reason, its acoustic velocity is not required to be a value different from the acoustic velocity of a subject (organism) as described in embodiment 1, and it may be set to a value that is virtually the same as the acoustic velocity of the subject so that no limitation is given to the acoustic velocity. Moreover, although the acoustic impedance (density x acoustic velocity) of shape-changing layer 120 is desirably set to a value close to the value (about 1.4 to 1.6 MRayls) of acoustic lens 180, the acoustic impedance may be set to a value between that of ultrasonic resonator 110 and that of the subject.

In this manner, ultrasonic transducer 300 of the present embodiment is provided with acoustic lens 180 for focusing ultrasonic waves placed on the subject side of shape-changing layer 120. In the same manner as in embodiment 1, by allowing the voltage to apply to shape-changing layer 120 to be variably changed, the shape of shape-changing layer 120 is changed, with the result that the curved surface of acoustic lens 180 placed on the front face of shape-changing layer 120 is changed. Since an ultrasonic beam to be transferred through acoustic lens 180 can be variably changed desirably, the ultrasonic beam can be focused linearly over a wide range in the depth direction of an ultrasonic image. As a result, an ultrasonic image with high resolution can be obtained, and by variably changing the voltage to apply to shape-changing layer 120 at high speeds, the ultrasonic image is allowed to have high resolution in real time.

Furthermore, the physically deformed portions are limited to only shape-changing layer 120 and acoustic lens 180, with ultrasonic resonator 110, backing material 130 or an acoustic matching layer to be installed, if necessary, being secured without being changed. For this reason, with respect to resonator 110, backing material 130 or the acoustic matching layer to be installed, if necessary, since no limitations are required for materials for use in deformation, it is possible to provide an ultrasonic transducer 300 with high performances.

Additionally, in the present embodiment, the explanation has been given by exemplifying a structure in which shape-changing layer 120 has a convex surface shape in its surface shape on the subject side; however, in addition to this structure, a structure may be proposed in which the surface shape on the subject side of shape-changing layer 120 is formed into a concave surface shape based upon relationships among shape-changing layer 120, acoustic lens 180 and the subject, and the same effects can be obtained.

Additionally, in the present embodiment, the explanation has been given by exemplifying a structure in which ultrasonic resonator 110 is a single unit; however, another structure may be proposed in which ultrasonic resonators 110 are arranged one-dimensionally or in which shape-changing layers 120 are arranged two-dimensionally, and the same effects can be obtained.

Moreover, the explanation has been given by exemplifying a structure in which ultrasonic resonator 110 is formed on the surface of shape-changing layer 120 on the subject side in a flat shape with an even thickness; however, even in the case of ultrasonic resonator 110 with an uneven thickness, or even in the case when it is formed on shape-changing layer 120 on the subject side, with a convex surface shape or a concave surface shape, the same effects can be obtained.

(Embodiment 4)

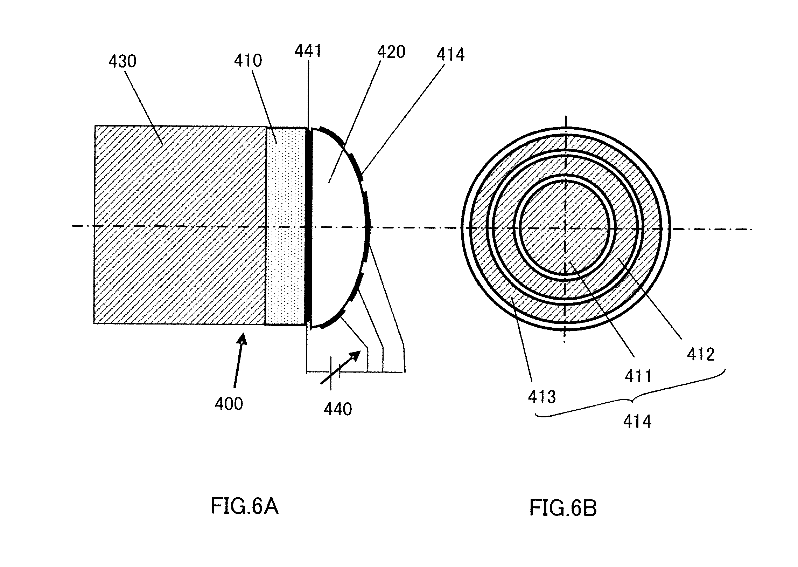

FIG.6 is a drawing that shows a schematic structure of an ultrasonic transducer in accordance with embodiment 4 of the present invention, and FIG.6A is a cross-sectional view thereof, and FIG.6B is a front view thereof.

As shown in FIG.6A, ultrasonic transducer 400 is provided with ultrasonic resonator 410, shape-changing layer 420, backing material 430, electrode 441 disposed on ultrasonic resonator 410 side of shape-changing layer 420, electrode 414 composed of a plurality of electrodes 411 to 413 that are formed on the convex shaped surface side of shape-changing layer 420, and a variable power supply 440 that applies electrical signals between electrode 441 and plural electrodes 411 to 413.

Ultrasonic transducer 400 is provided with electrode 414 composed of a plurality of electrodes 411 to 413.

As shown in FIG.6B, with respect to plural electrodes 411 to 413 formed on the convex shaped surface side of shape-changing layer 420, electrode 411 is placed at a leading position of the convex shaped surface of shape-changing layer 420, and electrodes 412 and 413 are disposed as concentric circles centered on electrode 411.

Variable power supply 440 adjusts an electrical signal (voltage in this case) to be applied between electrode 441 and plural electrodes 411 to 413. Variable power supply 440 can variably change voltages to be respectively applied to electrodes 411, 412 and 413 independently as well as desirably.

In the above-mentioned structure, variable power supply 440 applies a predetermined voltage between, for example, electrode 441 of shape-changing layer 420 and electrode 411 opposed thereto. Shape-changing layer 420 in the opposed region is changed in its shape, while shape-changing layer 420 in regions of the other electrodes 412 and 413 is not subjected to a force that causes a shape change independently so that it tries to maintain its shape (of shape-changing layer 420, although only the peripheral portion of the region opposed to electrode 411 whose shape is changed is subjected to the influences, the other portions are basically allowed to maintain their shapes). In this manner, the curved surface in the region of electrode 411 can be deformed. In the same manner, voltages to apply to electrodes 412 and 413 are respectively variably changed, the corresponding regions of shape-changing layer 420 can be changed desirably. Thus, shape-changing layer 420 is allowed to have a function of the acoustic lens so that, by variably changing its shape, an ultrasonic beam can be focused with a desired distance.

In accordance with the structure of FIG.6, not only a curved surface with a curvature simply having one center point, but also curved surfaces having various shapes, such as an aspheric surface, can be formed; therefore, a desired ultrasonic beam can be variably changed at high speeds by variably changing a voltage to be applied to shape-changing layer 420.

Additionally, in the present embodiment, a plurality of electrodes 411, 412, and 413 are formed on the convex shaped surface side; however, electrode 441 may be formed by using a conductive material having elasticity, or by forming cuts within the electrode surface in a spiral shape or the like, a deformable structure is provided so that the positional relationship may be replaced.

(Embodiment 5)

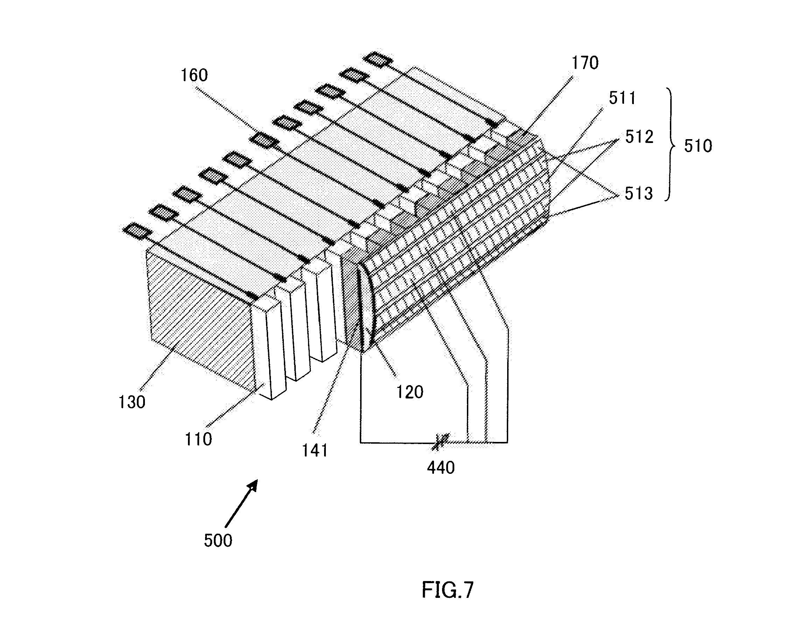

FIG.7 is a perspective view that shows a schematic structure of an ultrasonic transducer in accordance with embodiment 5 of the present invention. Those components having the same structures as those shown in FIG.4 are indicated by the same reference numerals, and overlapping explanations will not be repeated.

As shown in FIG.7, in place of electrode 142 on the convex shaped surface side of shape-changing layer 120 of ultrasonic transducer 200 of FIG.4, ultrasonic transducer 500 is provided with electrode 510 composed of a plurality of electrodes 511 to 513.

Electronic scanning-type ultrasonic transducer 500 is provided with shape-changing layer 120 placed on the front face of acoustic matching layer 170 that is formed on the subject side of each of a plurality of ultrasonic resonators 110 that are aligned. Electrode 141 is formed on one of surfaces of shape-changing layer 120, and on the other convex shaped surface side, electrode 510 composed of a plurality of electrodes 511 to 513 are placed in a band form in the aligning direction of ultrasonic resonators 110. Shape-changing layer 120 has a function of the acoustic lens so that an ultrasonic beam in a direction orthogonal to the aligning direction of ultrasonic resonators 110 is focused.

In the above-mentioned arrangement, variable power supply 440 applies a voltage, for example, to a portion of electrode 511 opposed to electrode 141 of shape-changing layer 120. Shape-changing layer 120 within the opposed region is deformed so that the shape of the surface (curved surface individually) of the surface of the corresponding portion of electrode 511 is changed. In the same manner, by variably changing voltages to apply to electrodes 512 and 513 respectively, the corresponding regions of shape-changing layer 120 can be desirably changed.

In the same manner as in the device having the structure of FIG.4, the structure of FIG.7 can form not only a curved surface with a curvature simply having one center point, but also curved surfaces having various shapes, such as an aspheric surface. Ultrasonic transducer 500 desirably adjusts voltages to be applied between electrode 141 and plural electrodes 511 to 513 so that upon diagnosing a concerned region, the shape of shape-changing layer 120 is changed so as to focus an ultrasonic beam to the corresponding region; thus, it is possible to variably change a desired ultrasonic beam at high speeds.

In this manner, operations that variably change the focusing position of an ultrasonic beam desirably by deforming the shape of shape-changing layer 120 are in cooperation with electronic controlling processes in the direction of aligned ultrasonic resonators 110. By using this cooperative control, it is possible to obtain an ultrasonic image with by far higher resolution.

In this case, the explanation has been given by exemplifying a structure in which plural electrodes 511, attached to shape-changing layer 120, are formed in a direction orthogonal to the direction of aligned ultrasonic resonators 110, and in addition to this, another arrangement may be proposed in which a plurality of electrodes 511 are formed in the direction of aligned ultrasonic resonators 110, and by deforming shape-changing layer 120 only within a desired position or region of ultrasonic resonators 110 in the aligning direction, the focusing position of an ultrasonic beam can be variably changed. Moreover, still another arrangement in which a plurality of electrodes 511 divided two dimensionally are installed can also provide the same effects.

(Embodiment 6)

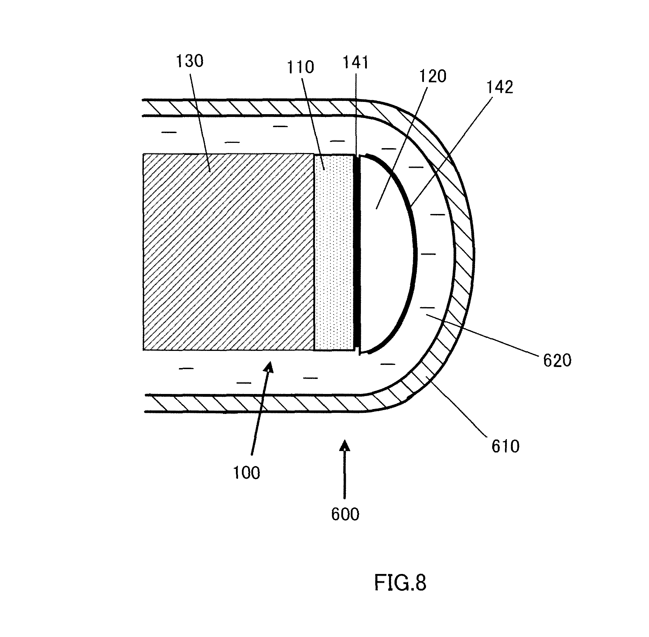

FIG.8 is a cross-sectional view that shows a schematic structure of an ultrasonic probe in accordance with embodiment 6 of the present invention. Those components that are the same as those of FIG.3 are indicated by the same reference numerals, and overlapping explanations will not be repeated.

As shown in FIG.8, ultrasonic probe 600 is provided with a case 610 having a cylindrical shape, ultrasonic transducer 100 enclosed in case 610, and an ultrasonic wave propagating medium 620 that is filled between the inside of case 610 and ultrasonic transducer 100.

Ultrasonic transducer 100 has the same structure as that explained in embodiment 1, and its functions are also the same. In place of ultrasonic transducer 100 of embodiment 1, ultrasonic transducers 200, 300, 400, and 500 of embodiments 2 to 5 may be applied.

Case 610 is directly or indirectly made in contact with a subject. As case 610, a material having an acoustic impedance close to that of the subject (for example, polyethylene, polymethylpentene, or the like) is used.

As ultrasonic wave propagating medium 620, a material having an acoustic impedance close to that of the subject (for example, 1, 3-butanediol or oil-based liquid) is used in the same manner as in case 610.

Ultrasonic waves, generated by ultrasonic resonators 110 of ultrasonic transducer 100, are allowed to permeate through shape-changing layer 120, ultrasonic wave propagating medium 620 and case 610, and transmitted to a subject. Ultrasonic waves reflected from the subject are again transmitted through a passage reversed to that of transmission, and received by ultrasonic resonators 110.

Ultrasonic probe 600 includes this ultrasonic transducer 100 in case 610, with ultrasonic wave propagating medium 620 interposed therebetween.

In accordance with the present embodiment, upon applying a voltage to shape-changing layer 120 so as to change the shape of shape-changing layer 120, it is possible to prevent the deformation of shape-changing layer 120 from being disturbed, which is different from an arrangement in which shape-changing layer 120 is directly made in contact with the subject, and used; thus, it becomes possible to variably change a desired ultrasonic beam at high speeds so as to be desirably formed.

Moreover, ultrasonic transducer 100 may be applied to ultrasonic probe 600 in which it is mechanically rotated, reciprocally moved, or rocked so as to carry out a scanning process.

Additionally, in the present embodiment, the explanation has been given by exemplifying a structure in which ultrasonic resonator 110 is a single unit; however, another structure may be proposed in which shape-changing layer 120 is formed in a structure in which ultrasonic resonators 110 are arranged one-dimensionally, or two-dimensionally, and the same effects can be obtained.

Moreover, the explanation has been given by exemplifying a structure in which ultrasonic resonator 110 is formed on the surface of shape-changing layer 120 on the subject side in a flat shape with an even thickness; however, even in the case of ultrasonic resonator 110 with an uneven thickness, or even in the case when it is formed on shape-changing layer 120 on the subject side, with a convex surface shape or a concave surface shape, the same effects can be obtained.

Additionally, in each of the embodiments, the explanations have been given by exemplifying a structure in which the surface shape of shape-changing layer 120 is a single curved surface; however, even in the case of a structure in which the curved surface of the surface shape of shape-changing layer 120 is an aspheric surface, or a non-curved surface, with its curvature radius varying depending on portions, or a shape in which only curvatures are aligned on a plane, such as a Fresnel lens, the same effects can be obtained.

On the other hand, in each of the embodiments, upon application of voltages to the respective electrodes, shape-changing layer 120 (420) has a different amount of change depending on places based upon its thickness even when the same voltage is applied thereto.

For example, in FIG.7 since the thickness is thinner in the peripheral end portion (region of electrode 513) of shape-changing layer 120 than that in the center portion (region of electrode 511) of shape-changing layer 120, the amount of change becomes greater, and since the thickness of electrode 513 in a region closer to electrode 512 side becomes closer to the thickness of the regions of electrode 512 and electrode 513, the curvature radius between these regions becomes greater. In contrast, in the region of electrode 511, in the case when the edge portion of the end portion of shape-changing layer 120 is considered, since the portion is greatly curved locally in comparison with a state where the initial voltage has not been applied, the curvature radius becomes smaller.

In this manner, since the curvature radius is different between the center portion and the peripheral portion of shape-changing layer 120, it becomes difficult to form a focal point at one portion; however, in the case when the length of each of ultrasonic resonators 110 that are arranged is shortened by cutting the two ends thereof so that ultrasonic waves are transmitted centered on the central portion of shape-changing layer 120, the focal point can be more easily formed. Moreover, by providing a structure in which a plurality of ultrasonic resonators are initially arranged two-dimensionally in a matrix form, a plurality of focal points are obtained between the center portion and the peripheral portion upon transmitting ultrasonic waves.

Additionally, by applying a voltage partially in such a manner as to apply only to the center portion (region of electrode 511) of shape-changing layer 120 or only to the peripheral end portion (region of electrode 513) of shape-changing layer 120, or by applying respectively different voltages thereto, the amount of change can be desirably set for respective portions to consequently provide a plurality of focal points.

Moreover, it is needless to say that as the area occupied by each electrode is made smaller by further increasing the number of divisions of electrodes 142 and 510 formed on the substrate side of shape-changing layer 120, the shape of the surface on the subject side of shape-changing layer 120 can be controlled more precisely.

In this case, in each of the embodiments, the explanation has been given by exemplifying a structure in which electrode 142, 410 or 510 formed on the subject side of shape-changing layer 120 is directly made in contact with the subject; however, another structure is proposed in which an insulating member, such as a film, that follows the deformation of shape-changing layer 120 is placed between electrode 142 or 510 and the subject, and the same effects can be obtained.

Moreover, the explanation has been given by exemplifying a structure in which the shape of the subject side surface of shape-changing layer 120 is formed into a convex surface; however, in addition to this, another structure may be proposed in which the shape of the subject side surface of shape-changing layer 120 is formed into a concave surface shape by utilizing the relationship between acoustic velocities of shape-changing layer 120 and the subject, and the same effects can be obtained.

Furthermore, the explanation has been given by exemplifying a structure in which ultrasonic resonator 110 is formed on the surface of shape-changing layer 120 on the subject side in a flat shape with an even thickness; however, in addition to this, even in the case of ultrasonic resonator 110 with an uneven thickness, or even in the case when it is formed on shape-changing layer 120 on the subject side, with a convex surface shape or a concave surface shape, the same effects can be obtained.

(Embodiment 7)

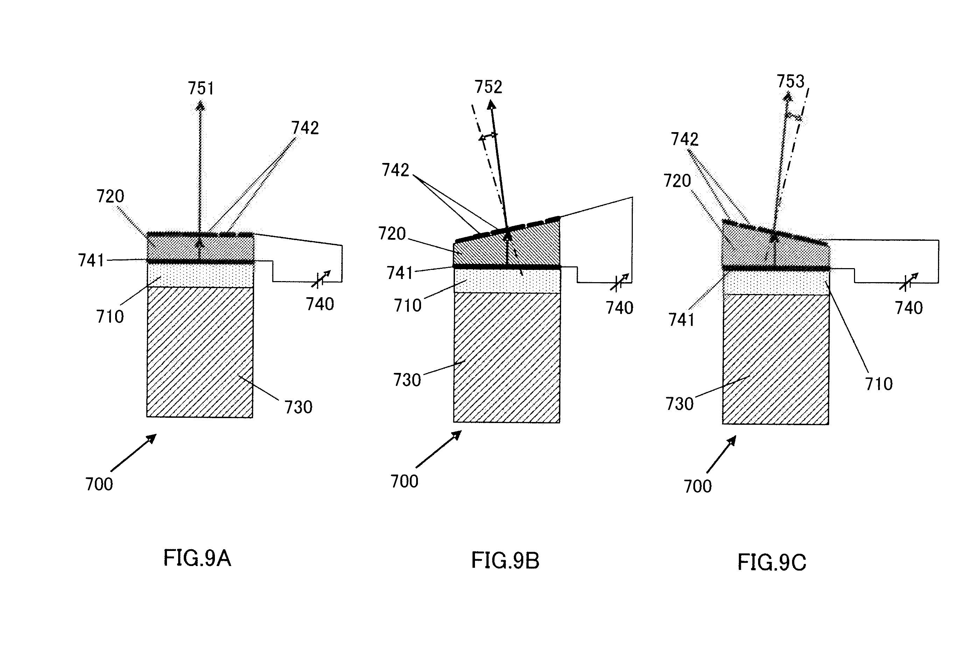

FIG.9 is a cross-sectional view that shows a schematic structure of an ultrasonic transducer in accordance with embodiment 7 of the present invention.

As shown in FIG.9, ultrasonic transducer 700 is provided with ultrasonic resonator 710, shape-changing layer 720, backing material 730, electrodes 741 and 742 that are disposed on the two surfaces of shape-changing layer 720, and a variable power supply 740 that applies an electrical signal between electrodes 741 and 742.

If necessary, one or more acoustic matching layers (not shown) may be installed between ultrasonic resonator 710 and shape-changing layer 720. These components other than shape-changing layer 720 respectively have the same functions as those explained in the prior art by reference to FIG.1.

Ultrasonic resonator 710 is made of a material such as a piezoelectric ceramic material of PZT (Lead Zirconate Titanate) based material, a piezoelectric single crystal such as PZN-PT or PMN-PT-based crystal, or a composite piezoelectric material that is a composite material between the above-mentioned material and a polymer. Moreover, in addition to these, a static capacity-type material, formed by applying a micromachining technique to a silicon semiconductor, may be used; thus, ultrasonic resonator 710 mentioned here is not intended to be limited by these, as long as it serves as an ultrasonic sensor that can transmit and receive ultrasonic waves.

A grounding electrode, not shown, is formed on the front face of ultrasonic resonator 710 in the thickness direction, and a signal electrode is formed on the backface thereof respectively. The two electrodes are respectively formed on the front face and backface of ultrasonic resonator 710, by using vapor deposition of gold or silver, a sputtering process, or a silver enameling process.

Shape-changing layer 720, which is placed on the front face in the thickness direction corresponding to the subject side (right side in FIG.3) relative to ultrasonic resonator 710, has functions for changing direction of an ultrasonic beam.

In the case when an acoustic velocity has a value that is different from the acoustic velocity (1540 m/s.) of a subject, for example, an organism, shape-changing layer 720 allows an ultrasonic beam to be refracted on the interface between shape-changing layer 720 and the subject so that each of ultrasonic beams 751, 752 and 753 can be variably changed in a desired direction. These functions are derived from the fact that, by making a difference in velocities between the acoustic velocity of shape-changing layer 720 and the acoustic velocity of the subject, ultrasonic waves are refracted on the interface thereof. By utilizing this refraction, the shape of shape-changing layer 720 is changed so that the directions of ultrasonic beams 751, 752 and 753 can be controlled.

In the present embodiment, shape-changing layer 720 is formed by using a material that can be variably changed in its shape on the subject side by applying an electrical signal. Electrodes 741 and 742 are formed on the two surfaces of shape-changing layer 720, and an electrical signal (in this case, voltage) is applied between electrodes 741 and 742. Variable power supply 740 adjusts the voltage to apply between electrodes 741 and 742. Thus, the shape of shape-changing layer 720 is changed so that the directions of ultrasonic beams 751, 752 and 753 are variably changed.

As the material of shape-changing layer 720, materials, such as an ionic polymer metal composite, a dielectric polymer, and a conductive polymer, whose polymer is deformed upon application of an electrical signal to its polymer are used.

The ionic polymer metal composite forms a polymer actuator made of an ion exchange resin with electrodes formed on its two surfaces, and ions inside the ion exchange resin are moved by an applied voltage so that the side on which the ions have been moved is swelled to make the polymer deformed. As the material of this polymer actuator, those materials, prepared by introducing a functional group, such as a sulfonic acid group and a carboxyl group, into a material such as polyethylene, polystyrene, and a fluorine resin, and polymeric materials in which, as disclosed by Patent Literature 3, a non-conductive polymer, such as polyvinyl chloride (PVC), polymethyl methacrylate (PMMA) and polyurethane, and an ionic material are contained, may be used.

The dielectric polymer is designed so that by applying a voltage between electrodes formed on the two surfaces of a polymer member, the polymer is compressed in its thickness direction by an electrostatic attracting force exerted between the electrodes, with the result that the polymer is expanded in its plane direction, and deformed. As this dielectric polymer, materials such as silicone rubber, polyurethane, and acrylic elastomer, may be used.

Moreover, in the case of the conductive polymer, electric terminals are drawn from a conductive polymer, and upon application of a voltage across the terminals, the conductive polymer between the electric terminals is contracted, while, upon turning the applied voltage off, the polymer is returned to its original state. As the dielectric conductive polymer, a polypyrrole resin or the like may be used.

In addition to the above-mentioned materials, any material may be used as long as its polymer material is deformed by an electrical signal, and the present invention is not intended to be limited by the above-mentioned materials.

In this case, the electricity of the electrical signal is mainly voltage-adjusted; however, this may be electric-current-adjusted. Moreover, the signal of the above-mentioned electrical signal is expressed as a signal by including arrangements in which its voltage is made variable, or applied timing or the like is made variable.

Backing material 730, which is designed so as to mechanically hold ultrasonic resonator 710 from the backface thereof, is disposed on the backface (lower side in FIG.9) in the thickness direction corresponding to the opposite side to shape-changing layer 720 relative to ultrasonic resonator 710, so as to exert a function for attenuating an unnecessary ultrasonic signal, if necessary.

The following description will discuss direction-variable operations of shape-changing layer 720.

In the case when the acoustic velocity of shape-changing layer 720 has a value that is different from the acoustic velocity (1540 m/s.) of a subject, for example, an organism, shape-changing layer 720 allows an ultrasonic beam to be refracted on the interface between shape-changing layer 720 and the subject so that each of ultrasonic beams 751, 752 and 753 can be variably changed in a desired direction. As the material for shape-changing layer 720, for example, polyethylene, polystyrene, polyvinyl chloride (PVC), polymethylmethacrylate (PMMA), or the like has an acoustic velocity faster than the acoustic velocity of an organism, that is, about 2000 m/s., while, in contrast, silicone rubber has an acoustic velocity slower than the acoustic velocity of an organism, that is, about 1000 m/s.

As has been conventionally known, the relationship of refraction among ultrasonic waves can be calculated based upon Snell's law. With respect to an ultrasonic wave directed on an interface between two media having acoustic velocities of C 1 and C2, the relationship of refraction of the ultrasonic wave that is made incident on the medium having acoustic velocity C2 from the medium having acoustic velocity C1 is represented by the following equation 2:

$$C1/\sin\theta i = C2/\sin\theta t \dots \text{(Equation 2)}$$

In this case, θi represents an angle at which the ultrasonic wave is made incident from a medium having an acoustic velocity of C1, and θt represents an angle at which it is made incident on a medium having an acoustic velocity of C2.

Upon comparison with cases in which acoustic velocity C1 of shape-changing layer 720 is derived from silicone rubber having a value of acoustic velocity of about 1000m/s, used as its dielectric polymer and in which it is derived from polyethylene vinyl chloride (PVC) or the like having a value of acoustic velocity of about 2000 m/s. used as its ionic polymer metal composite, with an organism having acoustic velocity C2 of about 1540 m/s. being used as a subject, the relationship of these can be calculated from the above-mentioned equation 2.

As shown in FIG.9A, in the case when no voltage is applied to shape-changing layer 720, an ultrasonic beam 751 to be propagated makes virtually right angles relative to the interface between shape-changing layer 720 and the subject, and is allowed to linearly proceed without being refracted.

In contrast, as shown in FIGS.9B and 9C, in the case when an electrical signal voltage is applied to electrodes 741 and 742 of shape-changing layer 720 to be controlled to allow shape-changing layer 120 to change so that a tilt is formed on the interface of the subject, the ultrasonic beams 752 and 753 are refracted to be variably changed in its proceeding direction.

For example, in the case when ultrasonic waves are transmitted from ultrasonic resonator 710 through the surface of shape-changing layer 720 on the subject side, and propagated to linearly proceed into shape-changing layer 720 so that the surface shape of shape-changing layer 720 is tilted by 20 degrees relative to the linearly proceeding direction of an ultrasonic beam 751, ultrasonic beams that are refracted and allowed to proceed through shape-changing layer 720, with acoustic velocity C 1 being set to about 1000 m/s. and about 2000 m/s., make respective angles θ of about 31.8 degrees and about 15.3 degrees. Therefore, by allowing shape-changing layer 720 to have a tilt angle of 20 degrees relative to the proceeding direction of ultrasonic beam 751 of FIG.9, the proceeding directions of ultrasonic beams 752 and 753 can be variably changed. In other words, the proceeding direction of an ultrasonic wave can be variably changed based upon the tilt angle of shape-changing layer 720 and the acoustic velocity of its material.

In this manner, by adjusting an electrical signal to apply to shape-changing layer 720, the tilt angle can be variably changed. Moreover, by controlling an electrical signal to apply to electrodes 741 and 742 of shape-changing layer 720 so that the tilt angle of shape-changing layer 720 is operated to be variably changed, a scanning process can be carried out, with the angles being variably changed as indicated by ultrasonic beams 751, 752 and 753.

The angles of ultrasonic beams 751, 752 and 753 to be used for scanning may be made wider than the above-mentioned angles, that is, about 31.8 degrees and about 15.3 degrees, with the applied voltage being variably changed to change the tilt angle of the surface of shape-changing layer 720.

Moreover, after transmitting and receiving an ultrasonic beam to and from a subject in a desired direction, by variably changing the voltage to apply to electrodes 741 and 742 of shape-changing layer 720 so as to change the shape, the direction of the ultrasonic beam is variably changed to be transmitted and received to and from the subject in a different direction, and by repeatedly carrying out these processes, a tomographic image of the subject can be formed. These controlling processes are carried out in a main unit section of the ultrasonic diagnostic apparatus, and realized.

As one of methods for variably changing the shape of the surface on the subject side of shape-changing layer 720, an electrode is formed on one of the surfaces of shape-changing layer, for example, a plurality of electrodes 742 are formed in FIG.9, and by variably changing an electrical signal, for example, a voltage to be applied to respective electrodes 742, the shape thereof can be variably changed. For example, in the case when an ionic polymer metal composite is used as shape-changing layer 720, by the applied voltage to electrodes 741 and 742, ions are transferred and the side having transferred ions is swelled to change its size of deformation so that, by variably changing the voltage to apply to respective electrodes 742, the amount of deformation in a portion of shape-changing layer 720 corresponding to the region that is placed on each of electrodes 742 can be changed. Thus, the surface on the subject side of shape-changing layer 720 can be variably changed desirably. The same effect can be obtained even in the case of using a dielectric polymer or a conductive polymer. However, it should be taken into consideration that electrodes 741 and 742, formed on shape-changing layer 720, need to have such a thickness (for example, as thin as possible so as to avoid influences) so as not to be influenced by propagation of ultrasonic waves.

As clearly indicated by the structure of FIG.9, since shape-changing layer 720 is made from a material through which ultrasonic waves are propagated, influences to be caused by the application of this material, for example, multiple reflections or the like, need to be kept minimum. For this reason, the acoustic impedance of shape-changing layer 720 is preferably designed to have about 1.4 to 1.65 Mrayl that is a value close to the acoustic impedance of the organism. However, even in the case when the acoustic impedance is far different from that of the subject, as long as the acoustic impedance is located between that of ultrasonic resonator 710 and that of the subject, it is possible to reduce multiple reflections by forming acoustic matching layers before and after shape-changing layer 720. For this reason, the acoustic impedance of shape-changing layer 720 may have a value located between that of ultrasonic resonator 710 and that of the subject.

In this manner, ultrasonic transducer 700 in accordance with the present embodiment is provided with ultrasonic resonator 710, shape-changing layer 720, disposed on the subject side of the ultrasonic resonator and a shape of which changes by an electrical signal, electrodes 741 and 742 that are disposed on the two surfaces of shape-changing layer 720, and variable power supply 740 that applies an electrical signal between electrodes 741 and 742, and variable power supply 740 controls the electrical signal to be applied between electrodes 741 and 742 to allow shape-changing layer 720 to change its surface shape on the subject side so that the direction of an ultrasonic beam that propagates through shape-changing layer 720 is variably changed desirably. The shape of shape-changing layer 720 is changed, with the result that the direction of an ultrasonic beam on the subject side of shape-changing layer 720 is consequently changed. Since the ultrasonic beam can be scanned at a desired angle, a two-dimensional ultrasonic image can be obtained, and since no mechanical scanning process as in the case of a conventional system is required, and since no motor is required, it becomes possible to obtain an ultrasonic image by using a small light-weight apparatus with superior operability.

Moreover, by changing the structure of electrode 742 or by forming a plurality of electrodes, the voltage to apply to each of the electrodes is controlled so that shape-changing layer 720 can be changed in its shape not only in one-dimensional directions, but also in two-dimensional directions. With this arrangement, it becomes possible to scan an ultrasonic beam three-dimensionally, and consequently to easily form a three-dimensional ultrasonic image.

Moreover, shape-changing layer 720 can be variably changed electrically, and since the variably changing time can be made faster mechanically, it becomes possible to obtain an ultrasonic image in real time. For this reason, by variably changing a voltage to be applied to shape-changing layer 720 at higher speeds, the ultrasonic image in real time is allowed to have higher resolution.

Furthermore, the deformation is made only in shape-changing layer 720, with ultrasonic resonator 710, backing material 730 and an acoustic matching layer to be installed, if necessary, being secured without being changed. For this reason, with respect to resonator 710, backing material 730 or the acoustic matching layer to be installed, if necessary, since no limitations are required for materials for use in deformation, it is possible to provide a transducer with high performances.

(Embodiment 8)

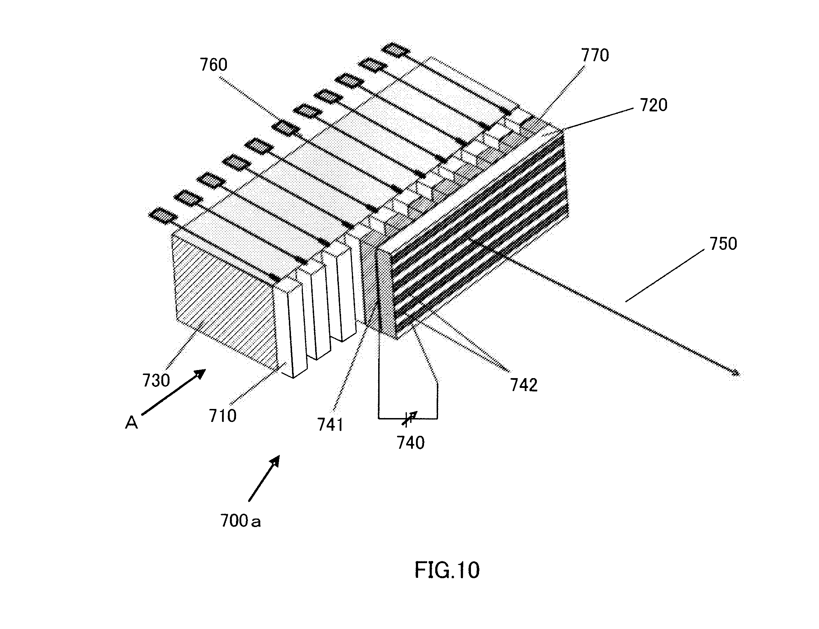

FIG.10 is a perspective view that shows a schematic structure of an ultrasonic transducer in accordance with embodiment 8 of the present invention. Those components that are the same as those shown in FIG.9 are indicated by the same reference numerals, and overlapping explanations will not be repeated. A view obtained in a direction of A of FIG.10 corresponds to the cross-sectional view of FIG.9.

As shown in FIG.10, ultrasonic transducer 700a is provided with a plurality of ultrasonic resonators 710 that are arranged, acoustic matching layer 770 that is disposed on the front face in the thickness direction corresponding to the subject side (right side of FIG.10) in association with respective ultrasonic resonators 710, backing material 730 that is disposed on ultrasonic resonators 710, if necessary, on the back face (left side of FIG.10) thereof in the thickness direction corresponding to the side opposite to acoustic matching layer 770, shape-changing layer 720 disposed on acoustic matching layer 770, electrodes 741 and 742 that are disposed on the two surfaces of shape-changing layer 720, and a variable power supply 740 that applies an electrical signal between electrodes 741 and 742. This structure is referred to as a so-called electron scanning-type ultrasonic transducer.

Each ultrasonic resonator 710 is made of a material such as a piezoelectric ceramic material of PZT based material, a piezoelectric single crystal such as PZN-PT or PMN-PT-based crystal, or a composite piezoelectric material that is a composite material between the above-mentioned material and a polymer. Moreover, in addition to these, a static capacity-type material, formed by applying a micromachining technique to a silicon semiconductor, may be used; thus, ultrasonic resonator 710 mentioned here is not intended to be limited by these, as long as it serves as an ultrasonic sensor that can transmit and receive ultrasonic waves.

A grounding electrode (not shown) is formed on the front face of ultrasonic resonator 710 in the thickness direction, and a signal electrode (not shown) is formed on the backface of ultrasonic resonator 710. The grounding electrode and the signal electrode are formed by plating gold or silver, vapor deposition, a sputtering process, or a silver enameling process. The signal electrode is formed by inserting and bonding signal electric terminal 760 formed by depositing a metal film, such as copper, onto an insulating film formed by a polymer material such as polyimide. The signal electrode is connected from the grounding electrode formed on ultrasonic resonators 710 to the grounding electric terminal (not shown), in the same manner as in signal electric terminal 760.

The following description will discuss operations of ultrasonic transducer 700a having the above-mentioned structure.

The signal electrode formed on each ultrasonic resonator 710 is electrically connected to one end of each of cables, not shown, through signal electric terminal 760 as well as through the grounding electrode of each ultrasonic resonator 710, and the other end of each of the cables is connected to a main unit section of an ultrasonic diagnostic apparatus, not shown, through a connector or the like. With this arrangement, a pulse voltage, prepared in the main unit section of the ultrasonic diagnostic apparatus, is applied to each ultrasonic resonator 710 so that ultrasonic waves are transmitted, and echoes of the received ultrasonic waves are converted into electrical signals and transmitted to the main unit section of the ultrasonic diagnostic apparatus. As this system that is mainly used, systems have been generally adopted in which a phase-controlling process is carried out by providing a time delay upon transmitting and receiving signals to each of arranged ultrasonic resonators 710 so that the ultrasonic beam is focused to a desired position so as to provide high resolution, or in which the ultrasonic beam is deflected so as to carry out a scanning process in a delta form.

The direction in which these ultrasonic beams are conversed at desired positions or in which the directions of ultrasonic beams are variably changed is controlled in association with the aligning direction of ultrasonic resonators 710.

Shape-changing layer 720 is designed such that, when the acoustic velocity of shape-changing layer 720 is a value that is different from the acoustic velocity (1540 m/s.) of a subject, for example, an organism, it allows an ultrasonic beam to be refracted on the interface between shape-changing layer 720 and the subject so that ultrasonic beam 750 can be variably changed in a desired direction. As explained in embodiment 17, by making a difference in velocities between the acoustic velocity of shape-changing layer 720 and the acoustic velocity of the subject, ultrasonic waves are refracted on the interface thereof so that by utilizing this refraction, the shape of shape-changing layer 720 is changed so that the direction of ultrasonic beam 750 is controlled. Shape-changing layer 120 has a function for variably changing the direction of an ultrasonic beam, and variably changes the direction of an ultrasonic beam in a direction orthogonal to the aligning direction of ultrasonic resonators 110.

In the present embodiment, shape-changing layer 720 is formed by using a material that can be variably changed in its shape on the subject side by applying an electrical signal thereto. Electrodes 741 and 742 are formed on the two surfaces of shape-changing layer 720, and an electrical signal (in this case, voltage) is applied between electrodes 741 and 742. Variable power supply 740 adjusts the voltage to apply between electrodes 741 and 742. Thus, by variably changing the shape of shape-changing layer 720, the direction of ultrasonic beam 750 can be controlled.

A plurality of electrodes 742 are placed on the subject side of shape-changing layer 720. These electrodes 742 are extended in a direction in which ultrasonic resonators 710 are aligned, and each electrode 742 in a direction orthogonal to the aligning direction of ultrasonic resonators 710 is divided into plurality of portions. Voltages of electrical signals to be applied to respective electrodes 742 are variably changed, and by changing the applied voltages, the surface on the subject side of shape-changing layer 720 is deformed in a direction orthogonal to the aligning direction of ultrasonic resonators 710 so that it can be deformed from a state before deformation as shown in FIG.9A to states as shown in FIGS. 9B and 9C so as to be tilted.

Additionally, the present embodiment has a structure in which a plurality of electrodes 742 are disposed on the subject side of shape-changing layer 720; however, another structure in which a plurality of electrodes are disposed on the side opposite to the subject side may be used.

Moreover, the present embodiment has a structure in which electrodes 742, formed on one of the surfaces of shape-changing layer 720, are extended in the aligning direction of ultrasonic resonators 710; however, by using a structure in which electrodes 742 are two-dimensionally arranged, the voltages to apply to these electrodes are controlled so that the direction of an ultrasonic beam can be variably controlled two-dimensionally. For example, the directions of ultrasonic beams on two ends of each of arranged ultrasonic resonators 710 may be made respectively different directions. Moreover, in an attempt to extract a concerned region of a diagnosis image, this operation is available by variably changing the direction of ultrasonic beam 750, without the necessity of moving ultrasonic transducer 700.

By using a material whose acoustic velocity is different from the acoustic velocity of a subject for shape-changing layer 720, the surface of shape-changing layer 720 is tilted so that ultrasonic beam 750 is variably changed in a direction orthogonal to the aligning direction of ultrasonic resonators 710 so as to carry out a scanning process.

For example, in the case when an ionic polymer metal composite is used for shape-changing layer 720, ions are transferred by the voltage applied to electrodes 741 and 742 so that the side having transferred ions is swelled to cause a change in the size of a polymer to be deformed; therefore, by variably changing the voltage to apply to respective electrodes 742, the amount of deformation of a portion of shape-changing layer 720 corresponding to a region positioned on each electrode 742 can be changed; thus, it becomes possible to variably change the surface on the subject side of shape-changing layer 720 desirably. This effect can also be obtained even in the case when a dielectric polymer or a conductive polymer is used. It should be taken into consideration that electrodes 741 and 742, formed on shape-changing layer 720, need to have such a thickness so as not to be influenced by propagation of ultrasonic waves.

Moreover, as clearly indicated by the structure of FIG.10, since shape-changing layer 720 is made from a material through which ultrasonic waves are propagated, influences to be caused by the application of this material, for example, multiple reflections or the like, need to be kept minimum. For this reason, the acoustic impedance of shape-changing layer 720 is preferably designed to have about 1.4 to 1.65 Mrayl that is a value close to the acoustic impedance of the organism.

In this manner, by allowing shape-changing layer 720 to be deformed in a direction orthogonal to the aligning direction of ultrasonic resonators 710, the direction of ultrasonic beam 750 can be variably scanned so that, even in a direction orthogonal to the aligning direction of ultrasonic resonators 710, an ultrasonic image can also be obtained, and by using this image in combination with the ultrasonic image acquired by an electron scanning process in the aligning direction of ultrasonic resonators 710, it is possible to obtain a three-dimensional ultrasonic image.

The main unit section of the ultrasonic diagnostic apparatus or the like controls a voltage to be applied to each of electrodes on the two surfaces of shape-changing layer 720 so as to variably change the direction of ultrasonic beam 750. The main unit section carries out a phase-controlling process, with a time delay being provided upon transmitting and receiving signals to and from groups of arranged ultrasonic resonators 710, so that the ultrasonic beam is transmitted and received. The main unit section carries out the scanning process, with the phase-controlling process being sequentially switched on an electronic basis, so that one cross section of a desired ultrasonic tomographic image can be obtained. Thereafter, the main unit section applies a controlled voltage to each of the electrodes of shape-changing layer 720, and controls so as to variably change the ultrasonic beam direction in a direction orthogonal to arranged ultrasonic resonators 710. By carrying out these scanning processes repeatedly, it is possible to achieve a three-dimensional ultrasonic image. These controlling processes can be easily carried out by the main unit of the ultrasonic diagnostic apparatus, and the deforming speed of shape-changing layer 720 can be made faster so that it is possible to obtain a three-dimensional image in real time.

In contrast to a conventional arrangement in which an ultrasonic transducer, such as an electron scanning-type ultrasonic transducer having a plurality of ultrasonic resonators 710 arranged therein, is reciprocally moved, or moved so as to rock by using a motor or the like, the present embodiment does not require any driving system such as a motor, and any transmitting system that transmits the driving force, and the ultrasonic transducer needs not be moved, with only the deformation of shape-changing layer 720 being required. Since the apparatus has a small size with light weight, and since the electric terminals and cables to be connected thereto need not be moved, with no other mechanisms or the like being required, high advantages for reliability, such as a long service life, can be achieved.

(Embodiment 9)

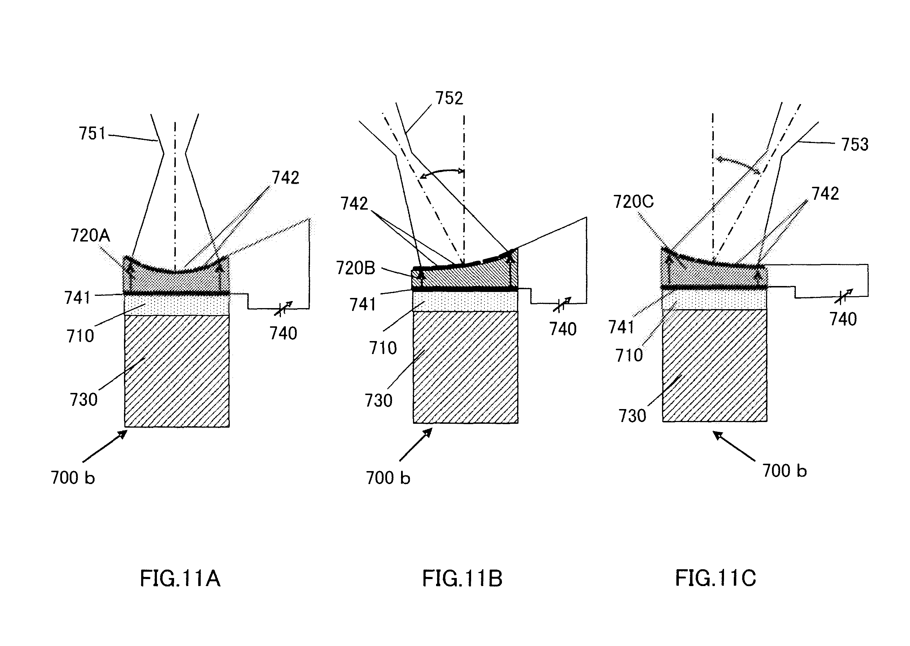

FIG.11 is a cross-sectional view that shows a schematic structure of an ultrasonic transducer in accordance with embodiment 9 of the present invention. Those components that are the same as those shown in FIG.9 are indicated by the same reference numerals, and overlapping explanations will not be repeated.

As shown in FIG.11, ultrasonic transducer 700b is provided with ultrasonic resonator 710, shape-changing layers 720A, 720B and 720C, backing material 730, electrodes 741 and 742 that are disposed on the two surfaces of each of shape-changing layers 720A, 720B and 720C, and a variable power supply 740 that applies an electrical signal between electrodes 741 and 742.

Moreover, if necessary, one or more acoustic matching layers (not shown) may be formed between ultrasonic resonator 710 and shape-changing layers 720A, 720B and 720C. These components other than shape-changing layers 720A, 720B and 720C have the same functions as those explained in the prior art of FIG. 1.

Ultrasonic resonator 710 is made of a material such as a piezoelectric ceramic material of PZT based material, a piezoelectric single crystal such as PZN-PT or PMN-PT-based crystal, or a composite piezoelectric material that is a composite material between the above-mentioned material and a polymer.

A grounding electrode, not shown, is formed on the front face of ultrasonic resonator 710 in the thickness direction, and a signal electrode, not shown, is formed on the backface thereof respectively. The two electrodes are formed on the front face and back face of ultrasonic resonator 710 by vapor deposition of gold or silver, sputtering, or an enameling process of silver.

Shape-changing layers 720A, 720B and 720C are disposed on the front face in the thickness direction corresponding to the subject side (upper side of FIG.11) relative to ultrasonic resonator 710, and have a function for variably changing the direction of an ultrasonic beam.

Functions of shape-changing layers 720A, 720B and 720C are the same as those of shape-changing layer 720 of FIG.9, and by utilizing the fact that the acoustic velocity of each of shape-changing layers 720A, 720B and 720C is different from the acoustic velocity of the subject, the direction of an ultrasonic beam is variably changed.

Moreover, to shape-changing layers 720A, 720B and 720C of the present embodiment, another function for allowing an ultrasonic beam to be focused to a desired depth of the subject is added. The explanation will be given in detail as follows:

As shown in FIGS.11A to 11C, the surface state on the subject side of each of shape-changing layers 720A, 720B and 720C is set to a curved surface shape.

As shown in FIG.11A, in the case when ultrasonic beam 751 is allowed to linearly proceed to be focused at a desired depth, the surface on the subject side of shape-changing layer 720A can be formed into a shape with a single curvature radius. With respect to the curved surface shape of the surface of shape-changing layer 720A, by forming it into a concave surface shape or a convex surface shape by utilizing a difference between the acoustic velocity of shape-changing layer 720A and the acoustic velocity of the subject, the ultrasonic beam can be focused. For example, in the case when the acoustic velocity of shape-changing layer 720A is a value slower than the acoustic velocity of the subject, the shape is formed into a convex surface in an attempt to focus the ultrasonic beam, and in contrast, in the case when the acoustic velocity of the shape-changing layer is faster, the shape is formed into a concave surface. These functions are the same as those contents explained in detail in embodiment 1.

As shown in FIGS.11B and 11C, in an attempt to variably change the directions of ultrasonic beams 752 and 753 and consequently to focus ultrasonic beams 752 and 753 to desired depths, the curved surfaces of the surfaces on the subject side of shape-changing layers 720B and 720C are formed into shapes whose curvature radii are gradually changed variably depending on positions.

These variable changes in the surface shapes on the subject side of shape-changing layers 720A, 720B and 720C can be obtained by dividing one of the electrodes of each shape-changing layer 720, that is, electrode 742 in this case, into a plurality of portions, and by controlling a voltage that is an electrical signal to apply to each of the electrodes. In order to variably change the focus and direction of an ultrasonic beam by deforming the shape of shape-changing layer 720 in this manner, it is necessary to carry out the deformation with high precision. In order to carry out the deformation with high precision, it needs to be taken into consideration that the number of plural electrodes 742 thus formed is increased, that the shape of each electrode is precisely formed and that a controlling process of a voltage to be applied to each of the electrodes is carried out with high precision.

For example, in the case when an ionic polymer metal composite is used as shape-changing layers 720A, 720B and 720C, by the applied voltage to electrodes 741 and 742, ions are transferred and the side having transferred ions is swelled to change the size of deformation of the polymer. Therefore, by variably changing the voltage to apply to respective electrodes 742, the amount of deformation in a portion of each of shape-changing layers 720A, 720B and 720C corresponding to the region that is placed on each of electrodes 142 can be changed.

Thus, the surface on the subject side of shape-changing layers 720A, 720B and 720C can be variably changed desirably. The same effect can be obtained even in the case of using a dielectric polymer or a conductive polymer. However, it should be taken into consideration that electrodes 741 and 742, formed on shape-changing layers 720A, 720B and 720C, need to have such a thickness so as not to be influenced by propagation of ultrasonic waves.

Although various materials are applicable to shape-changing layer 720, these materials have respective differences in applied voltage and amount of deformation; therefore, depending on materials to be used, it is necessary to optimize the voltage to apply.

Moreover, since shape-changing layers 720A, 720B and 720C are made from a material through which ultrasonic waves are propagated, influences to be caused by the application of this material, for example, multiple reflections or the like, need to be kept minimum. For this reason, the acoustic impedance of each of shape-changing layers 720A, 720B and 720C is preferably designed to have, for example, about 1.4 to 1.65 Mrayl that is a value close to the acoustic impedance of the organism.

Additionally, in the present embodiment, the explanation has been given by exemplifying a single structure of ultrasonic resonator; however, even in the case of an electron scanning-type structure with a plurality of ultrasonic resonators 710 arranged, or a so-called annular array-type structure in which ultrasonic resonators are arranged in a ring shape, the same effects can be obtained.

(Embodiment 10)

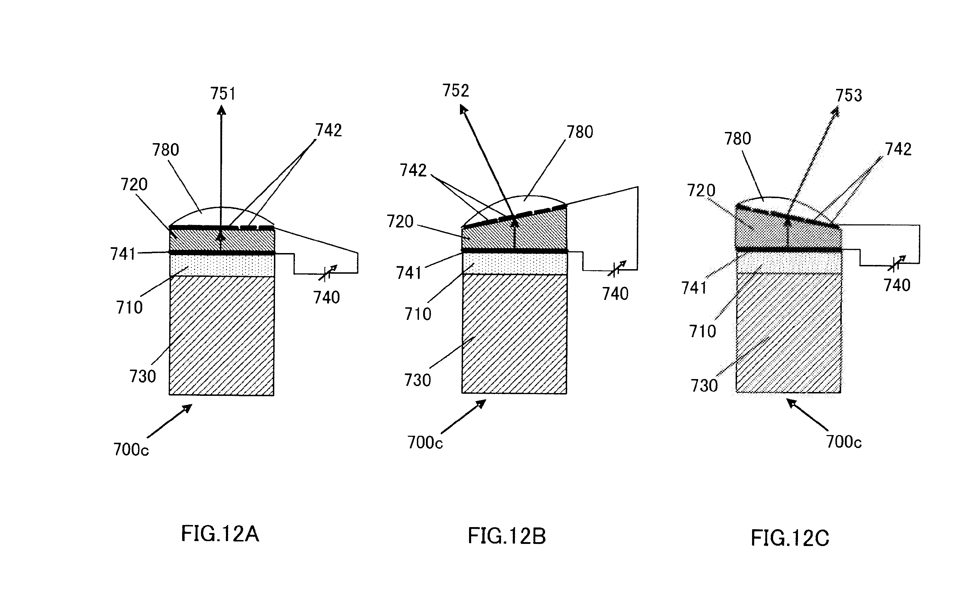

FIG.12 is a cross-sectional view that shows a schematic structure of an ultrasonic transducer in accordance with embodiment 10 of the present invention. Those components that are the same as those shown in FIG.9 are indicated by the same reference numerals, and overlapping explanations will not be repeated here.

As shown in FIG.12, ultrasonic transducer 700c is provided with ultrasonic resonator 710, shape-changing layer 720, backing material 730, electrodes 741 and 742 that are disposed on the two surfaces of shape-changing layer 720, a variable power supply 740 that applies an electrical signal between electrodes 741 and 742, and acoustic lens 780 that is disposed on the front face of shape-changing layer 720.

Moreover, if necessary, one or more acoustic matching layers (not shown) may be formed between ultrasonic resonator 710 and shape-changing layer 720. These components other than shape-changing layer 720 have the same functions as those explained in the prior art by reference to FIG. 1.

Ultrasonic resonator 710 is made of a material such as a piezoelectric ceramic material of PZT based material, a piezoelectric single crystal such as PZN-PT or PMN-PT-based crystal, or a composite piezoelectric material that is a composite material between the above-mentioned material and a polymer.

A grounding electrode, not shown, is formed on the front face of ultrasonic resonator 710 in the thickness direction, and a signal electrode is formed on the backface thereof respectively. The two electrodes are formed on the front face and backface of ultrasonic resonator 710 by vapor deposition of gold or silver, a sputtering process, or a silver enameling process.

Acoustic lens 780 allows an ultrasonic beam to be focused at a desired depth. Acoustic lens 780 is formed into a fixed shape by using a material such as, for example, silicone rubber.

As shape-changing layer 720, a material by which a shape on the subject side can be variably changed by using an electrical signal can be used. Electrodes 741 and 742 are formed on the two surfaces of shape-changing layer 720, and an electrical signal (in this case, a voltage) is applied between electrodes 741 and 742. Variable power supply 740 adjusts a voltage to be applied between electrodes 741 and 742.

Shape-changing layer 720 is formed by using a material, such as an ionic polymer metal composite, a dielectric polymer and a conductive polymer, whose polymer is deformed upon application of an electrical signal to the polymer. Shape-changing layer 720 is designed such that, when the acoustic velocity of shape-changing layer 720 is a value that is different from the acoustic velocity (1540 m/s.) of a subject, for example, an organism, it allows an ultrasonic beam to be refracted on the interface between shape-changing layer 720 and the subject so that ultrasonic beams 751, 752 and 753 can be variably changed in a desired direction. By making a difference in velocities between the acoustic velocity of shape-changing layer 720 and the acoustic velocity of the subject, ultrasonic waves are refracted on the interface thereof, and by utilizing this refraction, the shape of shape-changing layer 720 is changed so that the directions of ultrasonic beams 751, 752 and 753 are variably changed.

In this case, however, although the proceeding directions of ultrasonic beams 751, 752 and 753 can be variably changed, in the case when these are utilized to form an ultrasonic image, since ultrasonic beams 751, 752 and 753 are not focused, it is difficult to obtain high resolution. Therefore, acoustic lens 780 having a function for focusing ultrasonic beams 751, 752 and 752 is installed on the subject side of shape-changing layer 720.

With this arrangement, shape-changing layer 720 is allowed to have a function for variably changing the directions of ultrasonic beams 751, 752 and 753, while acoustic lens 780 has a function for focusing ultrasonic beams 751, 752 and 753. Thus, ultrasonic transducer 700c can obtain an ultrasonic image with high resolution.

Moreover, since shape-changing layer 720 is made from a material through which ultrasonic waves are propagated, influences to be caused by the application of this material, for example, multiple reflections or the like, need to be kept minimum. For this reason, the acoustic impedance of shape-changing layer 720 is preferably set to about 1.4 to 1.65 Mrayl that is a value close to the acoustic impedance of acoustic lens 780 or the subject (for example, organism).

As described above, by variably changing a voltage to be applied to shape-changing layer 720, the shape of shape-changing layer 720 is changed, with the result that the surface state on the subject side of shape-changing layer 720 is changed; therefore, it is possible to variably change the direction of an ultrasonic beam that is propagated through shape-changing layer 720 at a desired angle so as to carry out a scanning process. Moreover, since acoustic lens 780 for focusing the ultrasonic beam is attached to the front face of shape-changing layer 720, a two-dimensional ultrasonic image with high resolution can be obtained. Since no mechanical scanning process as in the case of a conventional system is required and since no motor is consequently required, it becomes possible to obtain an ultrasonic image by using a small light-weight apparatus with superior operability.

Moreover, by changing the structure of electrode 742, shape-changing layer 720 can be changed in its shape not only in one-dimensional directions, but also in two-dimensional directions. With this arrangement, it becomes possible to scan an ultrasonic beam three-dimensionally, and consequently to easily form a three-dimensional ultrasonic image.

Furthermore, shape-changing layer 720 can be variably changed electrically, and since its variably changing time can be made shorter in comparison with that of a mechanical system, it is possible to obtain an ultrasonic image in real time.

In the present embodiment, the explanation has been given by exemplifying a structure in which the acoustic impedance of shape-changing layer 720 has a value that is close to the impedance of acoustic lens 780 or that of the subject; however, in the case of using a material that makes the acoustic impedance value of shape-changing layer 780 different from that of the subject, an acoustic matching layer may be formed between shape-changing layer 720 and acoustic lens 780, and this structure also provides the same effects.

In the present embodiment, the explanation has been given by exemplifying a structure in which shape-changing layer 720 is directly formed on ultrasonic resonator 710; however, even in the case of a structure in which shape-changing layer 720 is not directly made in contact with the ultrasonic resonator, that is, is indirectly made in contact therewith, with an ultrasonic wave propagating medium or the like interposed therebetween, the same effects can be obtained.

Additionally, in the present embodiment, the explanation has been given by exemplifying a single structure of ultrasonic resonator; however, even in the case of an electron scanning-type structure with a plurality of ultrasonic resonators 710 arranged, or a so-called annular array-type structure in which ultrasonic resonators are arranged in a ring shape, the same effects can be obtained.

(Embodiment 11)

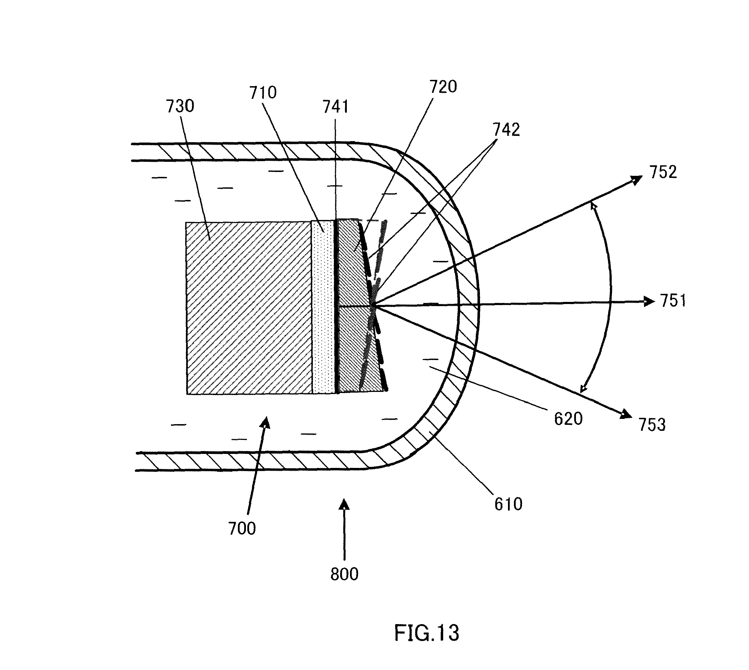

FIG.13 is a cross-sectional view that shows a schematic structure of an ultrasonic probe in accordance with embodiment 11 of the present invention. Those components that are the same as those shown in FIG.9 are indicated by the same reference numerals, and overlapping explanations will not be repeated here.

As shown in FIG.13, ultrasonic probe 800 is provided with a case 610 having a cylindrical shape, ultrasonic transducer 700 enclosed in case 610, and an ultrasonic wave propagating medium 620 that is filled between the inside of case 610 and ultrasonic transducer 700.

Ultrasonic transducer 700 has the same structure as that explained in embodiment 7, and its functions are also the same. In place of ultrasonic transducer 700 of embodiment 7, ultrasonic transducers 700a, 700b, and 700c of embodiments 7 to 10 may be applied.

Case 610 is directly or indirectly made in contact with a subject. As case 610, a material having an acoustic impedance close to that of the subject (for example, polyethylene, polymethylpentene, or the like) is used.

As ultrasonic wave propagating medium 620, a material having an acoustic impedance close to that of the subject (for example, 1, 3-butanediol or oil-based liquid) is used in the same manner as in case 610.

Ultrasonic waves, generated by ultrasonic resonators 710 of ultrasonic transducer 700, are allowed to permeate through shape-changing layer 720, ultrasonic wave propagating medium 620 and case 610, and transmitted to a subject. Ultrasonic waves reflected from the subject are again transmitted through a passage reversed to that of transmission, and received by ultrasonic resonators 710.

Ultrasonic probe 800 includes this ultrasonic transducer 700 in case 610, with ultrasonic wave propagating medium 620 interposed therebetween.

In accordance with the present embodiment, upon applying a voltage to shape-changing layer 720 so as to change the shape of shape-changing layer 720, it is possible to prevent the deformation of shape-changing layer 720 from being disturbed, which is different from an arrangement in which shape-changing layer 720 is directly made in contact with the subject, and used; thus, it becomes possible to variably change desired ultrasonic beams 751, 752 and 753 at high speeds so as to carry out a scanning process, and also to be desirably formed.

Moreover, ultrasonic transducer 700 may be applied to ultrasonic probe 800 in which it is mechanically rotated, reciprocally moved, or rocked so as to carry out a scanning process.

Additionally, in the present embodiment, the explanation has been given by exemplifying a structure in which ultrasonic resonator 710 is a single unit; however, another structure may be proposed in which shape-changing layer 720 is formed in a structure in which ultrasonic resonators 710 are arranged one-dimensionally, or two-dimensionally, and the same effects can be obtained.

In the above-mentioned embodiments 1 to 11, the explanation has been given by exemplifying a structure in which electrode 742 formed on the subject side of shape-changing layer 720 is directly made in contact with a subject; however, even in the case of a structure in which an insulating member, such as a film, that follows the deformation of shape-changing layer 720 is formed between electrode 742 and the subject for protection, the same effects can be obtained.

Moreover, in the above-mentioned embodiments, the explanation has been given by exemplifying a structure in which ultrasonic resonator 710 is formed on the surface of shape-changing layer 720 on the subject side in a flat shape with an even thickness; however, even in the case of ultrasonic resonator 710 with an uneven thickness, or even in the case when it is formed on the surface of shape-changing layer 720 on the subject side, with a convex surface shape or a concave surface shape, the same effects can be obtained.

Additionally, in each of the embodiments, the explanations have been given by exemplifying a structure in which the acoustic impedance of shape-changing layer 720 has a value close to the acoustic impedance of the subject; however, in addition to this, in the case of using a material that makes the value of the acoustic impedance of shape-changing layer 720 different from that of the subject, an acoustic matching layer may be formed between shape-changing layer 720 and the subject, and this structure also provides the same effects.

Moreover, in the above-mentioned embodiments, the explanation has been given by exemplifying a structure in which shape-changing layer 720 is directly formed on ultrasonic resonator 710; however, even in the case of a structure in which shape-changing layer 720 is not directly made in contact with the ultrasonic resonator, that is, is indirectly made in contact therewith, with an ultrasonic wave propagating medium or the like interposed therebetween, the same effects can be obtained.

(Embodiment 12)

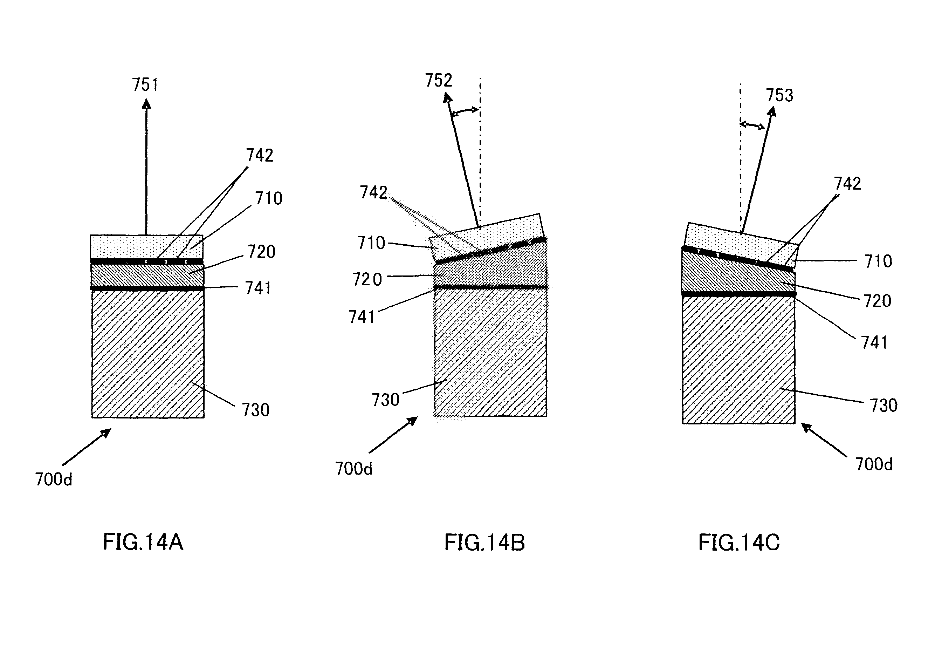

FIG.14 is a schematic cross-sectional view that shows a structure of an ultrasonic transducer in accordance with embodiment 12 of the present invention.

As shown in FIG.14, ultrasonic transducer 700d is made of a material such as a piezoelectric ceramic material of PZT (Lead Zirconate Titanate) based material, a piezoelectric single crystal such as PZN-PT (Lead Zinc Niobate Titanate) based crystal, or PMN-PT (Lead Magnesium Niobate Titanate) based crystal, or a composite piezoelectric material that is a composite material between the above-mentioned material and a polymer.

In addition to these, a static capacity-type material, formed by applying a micromachining technique to a silicon semiconductor, may be used; thus, ultrasonic resonator 710 mentioned here is not intended to be limited by these materials, as long as it serves an ultrasonic sensor that can transmit and receive ultrasonic waves.

The present embodiment proposes a structure which includes ultrasonic resonator 710, made of these materials, for transmitting and receiving ultrasonic waves, shape-changing layer 720 that is disposed on ultrasonic resonator 710 on the side opposite to the subject side (upper portion in FIG.14), that is on the backface side (lower portion of FIG.14) relative to transmitting and receiving directions of ultrasonic waves, and has a function for variably changing the direction of ultrasonic resonator 710, and backing material 730, placed on the backface of shape-changing layer 720, that holds ultrasonic resonator 710 and shape-changing layer 720, and has a function for, if necessary, attenuating unnecessary ultrasonic signals. Moreover, if necessary, one or more acoustic matching layers and an acoustic lens (not shown) may be formed on the surface on the subject side of ultrasonic resonator 710. Electrodes 741 and 742 are provided on the upper and lower surfaces of shape-changing layer 720. These components other than shape-changing layer 720 have the same functions as those explained in the prior art by reference to FIG. 1.

A grounding electrode, not shown, is formed on the front face of ultrasonic resonator 710 in the thickness direction, and a signal electrode is formed on the backface thereof respectively. The two electrodes are formed on the front face and backface of ultrasonic resonator 710 by vapor deposition of gold or silver, sputtering, or an enameling process of silver.

Shape-changing layer 720 is formed by using a material, such as an ionic polymer metal composite, a dielectric polymer and a conductive polymer, whose polymer is deformed by an electrical signal applied to the polymer.

The ionic polymer metal composite forms a polymer actuator made of an ion exchange resin with electrodes 741 and 742 formed on its two surfaces, and ions inside the ion exchange resin are moved by an applied voltage so that the side on which the ions have been moved is swelled to make the polymer deformed. The ionic polymer metal composite includes those materials, prepared by introducing a functional group, such as a sulfonic acid group and a carboxyl group, into a material such as polyethylene, polystyrene and a fluorine resin, and polymeric materials in which a non-conductive polymer, such as polyvinyl chloride (PVC), polymethyl methacrylate (PMMA) and polyurethane, and an ionic material are contained.

Electrodes 741 and 742 are formed on the two surfaces of a dielectric polymer member, and a voltage is applied between the electrodes. The dielectric polymer is compressed in its thickness direction by an electrostatic attracting force exerted between the electrodes, with the result that the polymer is expanded in its plane direction, and deformed. As this dielectric polymer, materials such as silicone rubber, polyurethane, and acrylic elastomer, may be used.

Moreover, in the case of the conductive polymer, electric terminals are drawn from a conductive polymer, and a voltage is applied between the electric terminals. The conductive polymer between the electric terminals is contracted, while, upon turning the applied voltage off, the polymer is returned to its original state. As the dielectric conductive polymer, a polypyrrole resin or the like may be used. In addition to the above-mentioned materials, any material may be used as long as its polymer material is deformed by an electrical signal, and the present invention is not intended to be limited by the above-mentioned materials.

In addition to these, not limited to the above-mentioned materials, any polymer actuator may be used, as long as it has a characteristic of being deformed upon application of an electrical signal to the polymer, and the same effects can be obtained.

In a state where a voltage, which serves as an electrical signal to apply to electrodes 741 and 742 of shape-changing layer 720, is not applied, the layer has a shape having no deformation with an even thickness, as shown in FIG.14A, and upon application of an electrical signal to ultrasonic resonator 710 in this state, ultrasonic resonator 710 oscillates to generate ultrasonic waves, and ultrasonic beam 751vf proceeds linearly, and is transmitted to the substrate side.

In a state shown in FIG.14A, a voltage serving as an electrical signal is applied to electrodes 741 and 742 of shape-changing layer 12. Thus, in the case when, for example, an ionic polymer metal composite is used, shape-changing layer 720 allows ion movements to occur in the polymer so that a polymer portion on the side on which ions have been transferred is expanded to cause a deformation in shape-changing layer 720. By utilizing this function, as shown in FIGS.14B and 14C, the voltage to apply to electrodes 741 and 742 of shape-changing layer 720 is variably adjusted. Thus, shape-changing layer 720 is deformed so that the surface on ultrasonic resonator 710 side can be tilted.

The voltage to apply to shape-changing layer 720 and the amount of deformation are virtually directly proportional to each other so that as the applied voltage becomes higher, the amount of deformation becomes greater. Thus, by adjusting the value of the applied voltage, the amount of deformation of shape-changing layer 720 can be adjusted. For this reason, ultrasonic resonator 710 formed on the front face of shape-changing layer 720 can be tilted following the deformation of shape-changing layer 720. As a result, ultrasonic beam 751 has its direction variably changed in the same manner as ultrasonic beams 752 and 753, as shown in FIGS. 14B and 14C.

In this manner, it becomes possible to deform shape-changing layer 720 by using a voltage. Since the directions of ultrasonic beams 751, 752 and 753 can be variably changed two-dimensionally or three-dimensionally, an ultrasonic image can be displayed two-dimensionally or three-dimensionally. In this case, a variable power supply used for applying an electrical signal to electrodes 741 and 742 formed on shape-changing layer 720 is not shown in FIGS.

More specifically, as one example of a method for variably changing the direction of the surface of ultrasonic resonator 710, the following method is proposed. With respect to electrodes formed on one of the surfaces of shape-changing layer 720, a plurality of electrodes 742 are formed as shown in FIG.14, and an electrical signal to apply to each of electrodes 742, for example, a voltage, is variably changed. Thus, as shown in FIGS. 14B and 14C, the thickness in the lateral direction of shape-changing layer 720 can be changed so that the transmitting and receiving directions of ultrasonic waves can be altered. For example, in the case when an ionic polymer metal composite is used as shape-changing layer 720, ions are moved by a voltage applied to electrodes 741 and 742, and the side on which the ions have been transferred has a change in its degree of deformation of the polymer. In this manner, by variably controlling the voltage to apply to respective electrodes 742, the amount of deformation of a portion of shape-changing layer 720 at a region positioned on each of electrodes 742. The direction of the surface of ultrasonic resonator 710 formed on the front face of shape-changing layer 720 can be variably changed desirably. This function can be exerted in the same manner also in the case of using a dielectric polymer or a conductive polymer.

Additionally, in order to deform shape-changing layer 720 with high precision as shown in FIG.14, a plurality of electrodes on one side of shape-changing layer 720 (electrodes 742 in this case) are formed, and a voltage to be applied to each of the electrodes is desirably adjusted by changing a pattern of electrodes.

Moreover, since shape-changing layer 720 has a structure in which it is formed between ultrasonic resonator 710 and backing material 730, the following method can be used. That is, so as to allow shape-changing layer 720 to have a function of backing material 730 for supporting ultrasonic resonator 710, an acoustic impedance close to that of backing material 730 is provided to shape-changing layer 720. Thus, attenuation of unnecessary ultrasonic signals is lowered, or problems, such as multiple reflections, caused by unnecessary ultrasonic waves inside shape-changing layer 720 can be eliminated. In the case of this structure, backing material 730 is set in a fixed state.

In FIG.14, the explanation has been given by exemplifying a structure in which backing material 730 is formed on the backface of shape-changing layer 720; however, in addition to this, another structure is proposed in which shape-changing layer 720 itself is formed into a shape (thickness) that can exert the function of the backing material, with a supporting base having a supporting function being formed on the backface of shape-changing layer 720, and this structure also provides the same effects.

As described above, by variably changing the voltage to apply to shape-changing layer 720, the shape of shape-changing layer 720 is changed so that the direction of ultrasonic resonator 710 formed on the front face thereof relative to the subject is consequently change. Thus, since the direction of an ultrasonic beam can be variably changed to a desired angle so as to carry out a scanning process, it is possible to obtain a two-dimensional ultrasonic image. Moreover, different from a conventional system, no mechanical scanning process is required and since no motor is consequently required, it becomes possible to obtain an ultrasonic image by using a small light-weight apparatus with superior operability.

Moreover, the structure of electrodes 742 in shape-changing layer 720 can be altered not only in one-dimensional direction, but also in two-dimensional directions. It is possible to scan the ultrasonic beam three-dimensionally, and a three-dimensional ultrasonic image can be easily achieved. Moreover, by variably changing shape-changing layer 720 electrically, the variably changing time can be made shorter in comparison with that of a mechanical system so that an ultrasonic image can be obtained in real time.

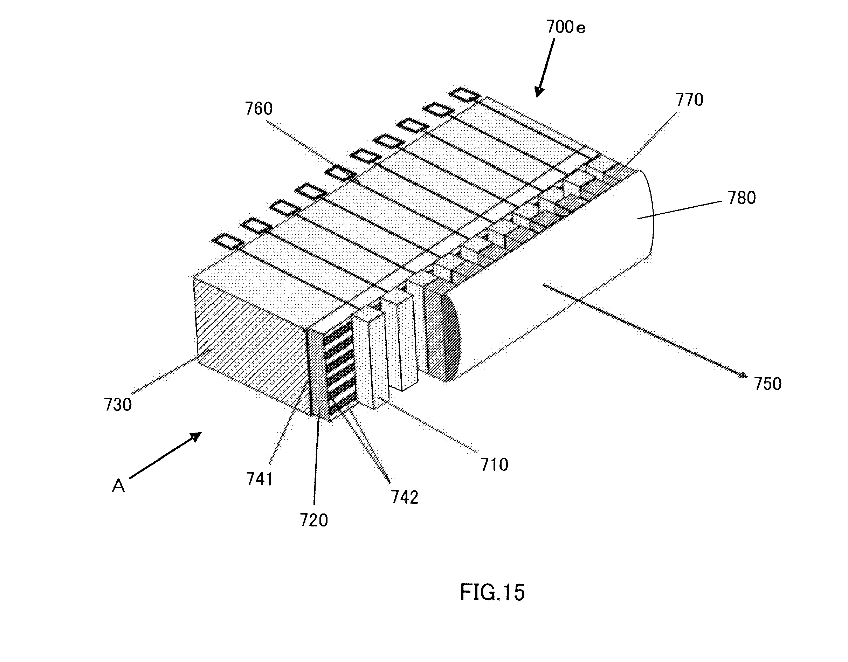

Although the explanation has been given by exemplifying a structure in which ultrasonic resonator 710 is a single unit, a plurality of ultrasonic resonators 710 may be installed as shown in FIG.15.

A drawing viewed in an A direction of FIG.15 is virtually the same as the drawing obtained by rotating each of FIG.14A to 14F by 90° anticlockwise. Those components having the same basic functions as those of FIG. 14 are indicated by the same reference numerals, and overlapping explanations will not be repeated here.

This ultrasonic transducer 700e is provided with a plurality of ultrasonic resonators 710 that are arranged and used for transmitting and receiving ultrasonic waves, acoustic matching layer 770 that is disposed on the front face in the thickness direction corresponding to the subject side (right side of FIG.15) in association with respective ultrasonic resonators 710, and used for improving performances, shape-changing layer 720 that is disposed, if necessary, on the backface in the thickness direction (left side in FIG.15) corresponding to the side opposite to acoustic matching layer 770 relative to ultrasonic resonator 710, backing material 730 that is further disposed on the backface thereof, and acoustic lens 780 that is disposed on acoustic matching layer 770, and focuses an ultrasonic wave so as to improve resolution. This structure is referred to as a so-called electron scanning-type ultrasonic transducer. Except for shape-changing layer 720, the functions of the respective components are the same as those explained in the prior art by reference to FIG.1.

In FIG.15, among components of ultrasonic transducer 700e, ultrasonic resonators 710 are formed by using a material such as a piezoelectric ceramic material of PZT based material, a piezoelectric member such as PZN-PT or PMN-PT-based material, or a composite piezoelectric material that is a composite material between the above-mentioned material and a polymer.

Moreover, in addition to these, a static capacity-type material, formed by applying a micromachining technique to a silicon semiconductor, may be used; thus, ultrasonic resonator 710 mentioned here is not intended to be limited by these, as long as it serves as an ultrasonic sensor that can transmit and receive ultrasonic waves.

A grounding electrode (not shown) is formed on the front face of ultrasonic resonator 710 serving as the subject side, and a signal electrode (not shown) is formed on the backface of ultrasonic resonator 710. The grounding electrode and the signal electrode are formed by plating gold or silver, vapor deposition, a sputtering process, or a silver enameling process.

Moreover, the signal electrode, formed on ultrasonic resonator 710, is prepared by inserting and bonding signal electric terminal 760 formed by depositing a metal film, such as copper, onto an insulating film formed by a polymer material such as polyimide. The signal electrode is connected from the grounding electrode formed on ultrasonic resonators 710 to the grounding electric terminal (not shown), in the same manner as in signal electric terminal 760.

The following description will discuss operations of ultrasonic transducer 700e having the above-mentioned structure.

The signal electrode formed on each ultrasonic resonator 710 is electrically connected to one end of each of cables, not shown, through signal electric terminal 760 as well as through the grounding electrode of each ultrasonic resonator 710, and the other end of each of the cables is connected to a main unit section of an ultrasonic diagnostic apparatus, not shown, through a connector or the like. With this arrangement, a regular pulse voltage, prepared in the main unit section of the ultrasonic diagnostic apparatus, is applied to each ultrasonic resonator 710. Ultrasonic transducer 700e transmits ultrasonic waves, and ultrasonic transducer 700e also converts echoes of ultrasonic waves that have been received into electrical signals, and transmits these to the main unit section of the ultrasonic diagnostic apparatus.

As this system, systems have been generally adopted in which a phase-controlling process is carried out by providing a time delay upon transmitting and receiving signals to each of arranged ultrasonic resonators 710 so that the ultrasonic beam is focused to a desired position relative to the direction of arranged ultrasonic resonators 710 so as to provide high resolution, or in which the ultrasonic beam is deflected so as to carry out a scanning process in a delta form. Electrodes 741 and 742 are formed on the upper and lower surfaces of shape-changing layer 720 placed between ultrasonic resonator 710 and backing material 730.

Shape-changing layer 720 is formed by using a material, such as an ionic polymer metal composite, a dielectric polymer and a conductive polymer, whose polymer is deformed by an electrical signal applied to electrodes 741 and 742 formed on the two surfaces of the polymer.

In a state where a voltage, which serves as an electrical signal to apply to electrodes 741 and 742 of shape-changing layer 720, is not applied, the layer has a shape having no deformation with an even thickness, as shown in FIG.15, and upon application of an electrical signal to ultrasonic resonator 710 in this state shown in FIG. 15, ultrasonic resonator 710 oscillates to generate ultrasonic waves, and ultrasonic beam 751 is linearly transmitted to the substrate side. In the state as shown in FIG.15, a voltage serving as an electrical signal is applied to electrodes 741 and 742 of shape-changing layer 720, and in this case, when an ionic polymer metal composite is used, shape-changing layer 720 allows ion movements to occur in the polymer so that a polymer portion on the side on which ions have been transferred is expanded to cause a deformation in shape-changing layer 720. By utilizing this function, as shown in FIGS. 14B and 14C serving as drawings viewed in an A direction of FIG.15, shape-changing layer 720 is deformed so that the surface can be tilted relative to a direction orthogonal to arranged ultrasonic resonators 710. By changing a pattern of electrodes 742, shape-changing layer 720 is also tilted not only in the direction orthogonal to arranged ultrasonic resonators 710, but also in the direction of the arranged ultrasonic resonators.

The voltage to apply to shape-changing layer 720 and the amount of deformation are virtually proportional to each other so that as the applied voltage becomes higher, the amount of deformation becomes greater. Thus, by adjusting the value of the applied voltage, the amount of deformation of shape-changing layer 720 can be adjusted. For this reason, ultrasonic resonator 710 formed on the front face of shape-changing layer 720 can be tilted following the deformation of shape-changing layer 720. As a result, ultrasonic beam 750 as shown in FIG.15 has its direction variably changed so as to carry out a scanning process in the same manner as ultrasonic beams 752 and 753, as shown in FIGS.14B and 14C.

As one method for variably changing the direction of the surface of ultrasonic resonator 710, a plurality of electrodes, that is, electrodes 742 in FIG.15, are formed one of the surfaces of shape-changing layer 720, and an electrical signal, for example, a voltage, to be applied to each of plural electrodes 742 is variably controlled. For example, in the case when an ionic polymer metal composite is used as shape-changing layer 720, ions are moved by the voltage applied to electrodes 741 and 742, and the side on which ions have been transferred is swelled so that the degree of deformation of the polymer is changed; thus, the voltage to apply to each of electrodes 742 is variably changed. With this arrangement, the amount of deformation of a portion of shape-changing layer 720 at a region positioned on each electrode 742 can be changed. Thus, the direction of the surface of ultrasonic resonator 710 formed on the front face of shape-changing layer 720 can be variably changed desirably. Even in the case of using a dielectric polymer or a conductive polymer, this effect can be obtained in the same manner.

Additionally, in order to deform shape-changing layer 720 with high precision as shown in FIG.14, a plurality of electrodes on one side of shape-changing layer 720 (electrodes 742 in this case) are formed, and a voltage to be applied to each of the electrodes is desirably adjusted by changing a pattern of electrodes.

Moreover, since shape-changing layer 720 has a structure in which it is formed between ultrasonic resonator 710 and backing material 730, the following method can be used. That is, so as to allow shape-changing layer 720 to have a function of backing material 730 for supporting ultrasonic resonator 710, an acoustic impedance close to that of backing material 730 is provided to shape-changing layer 720. Thus, attenuation of unnecessary ultrasonic signals is lowered, or problems, such as multiple reflections, caused by unnecessary ultrasonic waves inside shape-changing layer 720 can be eliminated. In the case of this structure, backing material 730 is set in a fixed state.

In FIG.15, the explanation has been given by exemplifying a structure in which backing material 730 is formed on the backface of shape-changing layer 720; however, in addition to this, another structure is proposed in which shape-changing layer 720 itself is formed into a shape (thickness) that can exert the function of the backing material, with a supporting base having a supporting function being formed on the backface of shape-changing layer 720, and this structure also provides the same effects.

As described above, by deforming shape-changing layer 720 in a direction orthogonal to the aligning direction of ultrasonic resonators 710, ultrasonic beam 750 is allowed to carry out a variable scanning process in a direction orthogonal to the aligning direction of ultrasonic resonators 710. Thus, since an ultrasonic image can be obtained also in a direction orthogonal to the aligning direction of ultrasonic resonators 71, a three-dimensional ultrasonic image can be obtained by combining this with an ultrasonic image acquired by an electron scanning process in the aligning direction of ultrasonic resonators 710.

By combinedly using the system for variably changing the direction of ultrasonic beam 750 by using shape-changing layer 720 and the system for variably changing ultrasonic transducer 700 by using a conventional motor or the like, the degree of freedom of the variably-changing process of the ultrasonic beam is further expanded.

Moreover, in a conventional system, ultrasonic transducer 700 is allowed to carry out reciprocal movements, rocking movements, or the like, by using a motor or the like. In contrast, in the present embodiment, neither a driving system, such as a motor, nor an axis for transmitting the driving force is required. Furthermore, in the present embodiment, since ultrasonic transducer 700e needs not be moved, with only shape-changing layer 720 being deformed, a small-size, light-weight system can be achieved, and electric terminals and cables or the like to be connected thereto need not be moved. For this reason, since no deformation is caused, and since mechanical structures are not required, the service life becomes longer with high reliability.

In the present embodiment, the explanation has been given by exemplifying a structure in which ultrasonic resonator 710 has an even thickness; however, in addition to this, even in the case of ultrasonic resonator 710 with an uneven thickness, or even in the case when it has a convex surface shape or a concave surface shape, the same effects can be obtained.

Moreover, in the present embodiment, the explanation has been given by exemplifying a structure in which shape-changing layer 720 is formed between ultrasonic resonator 710 and backing material 730; however, in addition to this, the position at which shape-changing layer 720 is formed is not particularly limited by this, and even in the case when it compatibly serves as the backing material, while being formed on the backface side of backing material 730, the same effects can be obtained as long as the corresponding structure can variably change the direction of ultrasonic resonator 710.

(Embodiment 13)

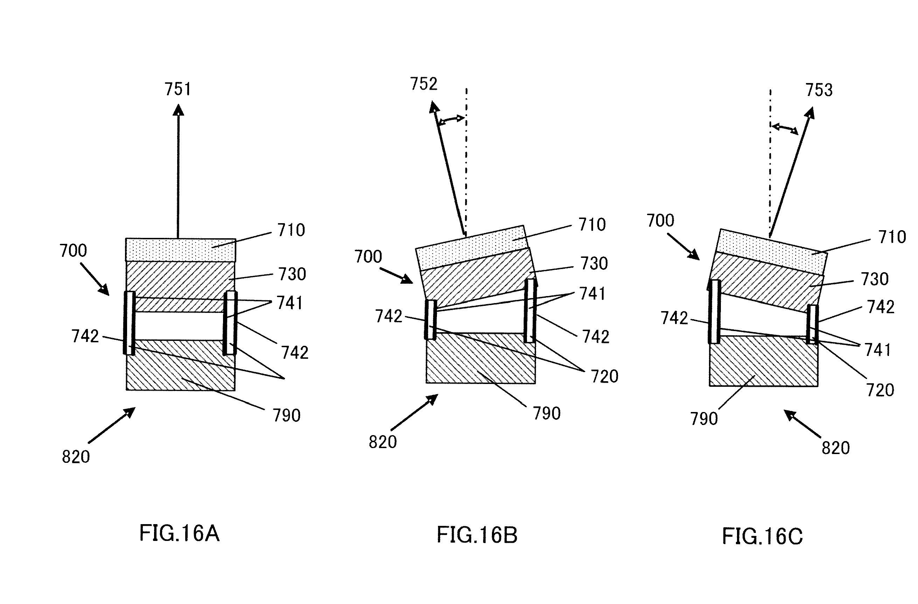

FIG.16 is a schematic cross-sectional view that shows an ultrasonic probe in accordance with embodiment 13 of the present invention. Those components that are the same as those shown in FIG.12 are indicated by the same reference numerals, and overlapping explanations thereof will not be repeated here.

As shown in FIG.16, transducer 700 of ultrasonic probe 820 is provided with ultrasonic resonator 710 that is made of a material such as a piezoelectric ceramic material of PZT based material, a piezoelectric single crystal such as PZN-PT or PMN-PT-based crystal, or a composite piezoelectric material that is a composite material between the above-mentioned material and a polymer, and backing material 730 that is disposed on the backface (lower side of FIG.16) on the side opposite to the subject side (upper side of FIG.16) in association with ultrasonic resonator 710, holds ultrasonic resonator 710, and has a function for, if necessary, attenuating unnecessary ultrasonic signals.

Moreover, ultrasonic probe 820 is provided with shape-changing layer 720 that forms one portion of backing material 730 of ultrasonic transducer 700, and has a function for driving ultrasonic resonator 710 and backing material 730, and fixed base 790 serving as a base to which one portion of shape-changing layer 720 is connected so as to drive ultrasonic resonator 710 and backing material 730. Electrodes 741 and 742 are formed on the two surfaces of shape-changing layer 720.

Moreover, in ultrasonic probe 820, if necessary, one or more acoustic matching layers and an acoustic lens (not shown) may be formed on the surface on the subject side of ultrasonic resonator 710.

Shape-changing layer 720 is formed by using a material, such as an ionic polymer metal composite, a dielectric polymer and a conductive polymer, whose polymer is deformed by an electrical signal to apply to electrodes 741 and 742 that are formed on the two surfaces of polymer.

As shown in FIG.16, two shape-changing layers 720 are installed on right and left sides of backing material 730 and fixed base 790.

In a state where a voltage, which serves as an electrical signal to apply to electrodes 741 and 742 of shape-changing layer 720, is not applied, the layer has a shape having no deformation with an even thickness, as shown in FIG.16A (in FIG.314, uniform length relative to transmitting and receiving directions of ultrasonic waves), and upon application of an electrical signal to ultrasonic resonator 710 in this state, ultrasonic resonator 710 oscillates to generate ultrasonic waves, and ultrasonic beam 751 is linearly transmitted to the substrate side. In a state shown in FIG.16A, a voltage serving as an electrical signal is applied to electrodes 741 and 742 of shape-changing layer 720. Thus, in the case when, for example, an ionic polymer metal composite is used, shape-changing layer 720 has such a function for allowing ion movements to occur so that shape-changing layer 720 is deformed. By utilizing this function, as shown in FIGS.16B and 16C, by applying different voltages to right and left shape-changing layers 720, deformations are caused with different thicknesses so that ultrasonic transducer 700 of ultrasonic resonator 710 and backing material 730 can be tilted. Thus, the direction of the surface of ultrasonic resonator 710 formed on the front face of backing material 730 can be tilted. In this case, fixed base 790 is secured so as not to be moved.

More specifically, in FIG.16, two shape-changing layers 720 are installed on the right and left sides of backing material 730. By individually adjusting voltages to apply to these two shape-changing layers 720, the amount of deformation can be adjusted. Ultrasonic transducer 700 constituted by ultrasonic resonator 710 and backing material 730 is driven to be tilted as shown in FIGS.16A to 16B and 16C. As shown in FIG.16, the direction of each of ultrasonic beams 751, 752 and 753 from ultrasonic resonator 710 can be variably changed so as to carry out a scanning process.

The voltage to apply to shape-changing layer 720 and the amount of deformation are virtually directly proportional to each other so that as the applied voltage becomes higher, the amount of deformation becomes greater. Thus, by adjusting the value of the applied voltage, the amount of deformation of shape-changing layer 720 can be adjusted.

As described above, in the present embodiment, by variably changing the voltage to apply to shape-changing layer 720, the shape of shape-changing layer can be changed. Since the direction of ultrasonic transducer 700 relative to the subject is changed, the direction of the ultrasonic beam can be variably changed with a desired angle so as to carry out a scanning process so that a two-dimensional ultrasonic image can be obtained.

Moreover, since a mechanical scanning process as in the case of a conventional system is not required, no motor is required so that it is possible to obtain an ultrasonic image by using a system with a small size and light weight having superior operability.

Furthermore, in the present embodiment, by changing a pattern structure of electrodes 742, shape-changing layer 720 can be changed not only in one-dimensional direction, but also in two-dimensional directions. In the same manner, by providing a structure in which an ultrasonic beam is used for a three-dimensional scanning process, it is possible to easily achieve a three-dimensional ultrasonic image.

In the present embodiment, since shape-changing layer 720 is variably changed electrically, a variably-changing time can be made faster in comparison with that of a mechanical system so that an ultrasonic image can be obtained in real time.

Additionally, shape-changing layer 720 of FIG. 16 is designed to have a structure in which the inside and outside of FIG.16 are provided with electrodes having the same functions; however, the setting method for electrodes is not limited by this. Moreover, electrodes may be formed not on the surface in a longitudinal direction of shape-changing layer 720, but on the surface on the backing material 730 side and the surface on the fixed base 790 side.

Moreover, in the case when electrodes are formed on the surface in the longitudinal direction of shape-changing layer 720, a reinforcing member that allows the deformation in the longitudinal direction to be preferentially carried out, with the deformation in directions toward the gap between the electrodes being limited, may be installed.

Furthermore, it is not necessary to form electrodes on a conductive polymer serving as a material for shape-changing layer 720, and when a voltage is applied across electric terminals in a structure in which the electric terminals are drawn from the conductive polymer, it is possible to initiate the deformation.

In the present embodiment, the explanation has been given by exemplifying a structure in which ultrasonic resonator 710 is a single unit; however, in addition to this, even in the case of a so-called one-dimensional, or two-dimensional array-type structure with a plurality of ultrasonic resonators 710 arranged, or a so-called annular array-type structure in which ultrasonic resonators are arranged in a ring shape, the same effects can be obtained.

Moreover, in the present embodiment, the explanation has been given by exemplifying a structure in which two shape-changing layers are formed on the fixed base and one portion of the side face of the backing material; however, even in the case of a structure in which one or three or more shape-changing layers are formed between the backing material and the fixed base, the same effects can be obtained.

Moreover, in the present embodiment, the explanation has been given by exemplifying a structure in which a plurality of shape-changing layers are formed on the fixed base and one portion of the side face of the backing material, with a space being formed between the fixed base and the backing material; however, in addition to this, even in the case of a structure in which a shape-changing layer is directly formed between the fixed base and the backing material, the same effects can be obtained.

(Embodiment 14)

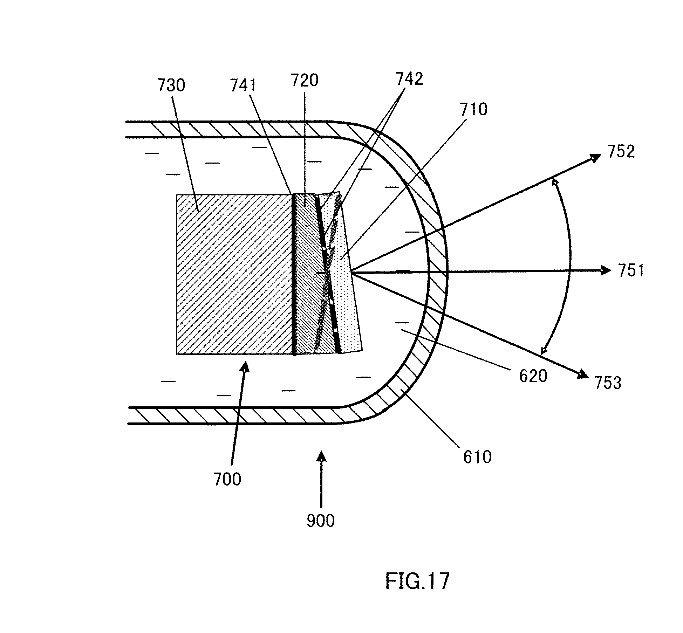

FIG.17 is a partial schematic cross-sectional view showing an ultrasonic probe in accordance with embodiment 14 of the present invention. Those components that are the same as those of FIG.16 are indicated by the same reference numerals, and overlapping explanations will not be repeated here.

As shown in FIG.17, ultrasonic transducer 700 of ultrasonic probe 900 is provided with ultrasonic resonator 710 that is made of a material such as a piezoelectric ceramic material of PZT based material, a piezoelectric single crystal such as PZN-PT or PMN-PT-based crystal, or a composite piezoelectric material that is a composite material between the above-mentioned material and a polymer. Moreover, ultrasonic transducer 700 is provided with shape-changing layer 720 that is disposed on the backface (left side of FIG.17) that is the side opposite to the subject side (right side of FIG.17) in association with ultrasonic resonator 710, and has a function for variably changing the direction of ultrasonic resonator 710, and backing material 730 that is disposed on the backface of shape-changing layer 720, holds ultrasonic resonator 710 and shape-changing layer 720, and has a function for, if necessary, attenuating unnecessary ultrasonic wave signals.

Moreover, if necessary, one or more acoustic matching layers and an acoustic lens (not shown) may be formed on the surface on the subject side of ultrasonic resonator 710. Electrodes 741 and 742 are provided on the upper and lower surfaces of shape-changing layer 720. These components other than shape-changing layer 720 have the same functions as those explained in the prior art.

The structure and functions of ultrasonic transducer 700 are the same as those explained in embodiments 12 and 13.

As shown in FIG.17, ultrasonic transducer 700 is enclosed in a case 610, and has a structure in which an ultrasonic wave propagating medium 620 such as a liquid is filled between case 610 and ultrasonic transducer 700. Case 610 is directly or indirectly made in contact with a subject. Ultrasonic waves, generated from ultrasonic resonator 710, are propagated through shape-changing layer 720, ultrasonic propagating medium 620 and case 610 to be transmitted to the subject, and ultrasonic waves reflected from the subject are again transmitted through a passage reversed to that of transmission, and received by ultrasonic resonators 710.

As case 610, a material having an acoustic impedance close to that of the subject, for example, polyethylene, polymethylpentene, or the like, is used. As ultrasonic wave propagating medium 620, a material having an acoustic impedance close to that of the subject, for example, 1, 3-butanediol or oil-based liquid, is used in the same manner as in the case of case 610.

Ultrasonic transducer 700 is enclosed in case 610; therefore, even upon applying a voltage to shape-changing layer 720 so as to change the shape of shape-changing layer 720, it is possible to prevent the deformation of shape-changing layer 720 from being disturbed, which is different from an arrangement in which shape-changing layer 720 is directly made in contact with the subject, and used. Thus, since the deformation of shape-changing layer 720 is not disturbed, it becomes possible to carry out a variable scanning process at high speeds. FIG.17 shows ultrasonic beams 751, 752 and 753 that are used for variable scanning processes at high speeds.

Moreover, ultrasonic transducer 700 may be applied to ultrasonic probe 900 in which it is mechanically rotated, reciprocally moved, or rocked so as to carry out a scanning process. In addition to ultrasonic transducer 700, that having the structure of embodiment 13 may be used.

Additionally, in the present embodiment, the explanation has been given by exemplifying a structure in which ultrasonic resonator 710 is a single unit; however, another structure may be proposed in which shape-changing layer 720 is formed in a structure in which ultrasonic resonators 710 are arranged one-dimensionally, or two-dimensionally, and the same effects can be obtained.

Moreover, in the present embodiment, the explanation has been given by exemplifying a structure in which ultrasonic resonator 710 is formed on the surface of shape-changing layer 720 on the subject side in a flat shape with an even thickness; however, in addition to this, even in the case of ultrasonic resonator 710 with an uneven thickness, or even in the case when it is formed on shape-changing layer 720 on the subject side, with a convex surface shape or a concave surface shape, the same effects can be obtained.

Although the above descriptions illustrate the present invention, they are exemplary only, and the scope of the present invention is not intended to be limited by these.

In the respective embodiments, the terms, the ultrasonic transducer and the ultrasonic probe, have been used; however, these are used only for convenience of explanation, terms, such as transducer, ultrasonic beam device, and the like, may be used.

The disclosures of Japanese Patent Application No. 2009-051314, filed on March 4, 2009, Japanese Patent Application No. 2009-051315, filed on March 4, 2009, and Japanese Patent Application No. 2009-054551, filed on March 9, 2009, including the specifications, drawings and abstracts, are incorporated herein by reference in their entirety.

Industrial Applicability

The ultrasonic transducer, ultrasonic probe and ultrasonic diagnostic apparatus in accordance with the present invention are applicable to various medical fields that carry out an ultrasonic diagnosis of a subject, such as a human body and the like, and industrial fields for the purposes of searching for internal damages or the like of a material and a structure.

Reference Signs List

100, 200, 300, 400, 500, 700, 700a, 700b, 700c, 700d, 700e Ultrasonic transducer
110, 410, 710 Ultrasonic resonator
120, 420, 720 Shape-changing layer
130, 430, 730 Backing material
140, 440, 740 Variable power supply
141, 142, 411 to 414, 441, 510, 511 to 513, 741, 742 Electrode
160, 760 Signal electric terminal
170, 770 Acoustic matching layer
180, 780 Acoustic lens
600, 800, 820, 900 Ultrasonic probe
610 Case
620 Ultrasonic propagating medium
790 Fixed base

**Claims**

1. An ultrasonic transducer comprising:

   an ultrasonic resonator which transmits and receives ultrasonic waves;
   a shape-changing layer, which is disposed on a subject side of the ultrasonic resonator, and a shape of which changes upon receipt of an electrical signal;

a control section which controls the electrical signal to apply to the shape-changing layer, wherein the control section changes the shape of the shape-changing layer by controlling the electrical signal to apply to the shape-changing layer so that the direction of ultrasonic waves is variably changed.

2. The ultrasonic transducer according to claim 1, further comprising:

an ultrasonic resonator array in which a plurality of ultrasonic resonators which transmit and receive ultrasonic waves are arranged;
a shape-changing layer which is disposed on a subject side of the ultrasonic resonator and a shape of which changes upon receipt of an electrical signal; and
a control section which controls the electrical signal to apply to the shape-changing layer, wherein the control section changes the shape of the shape-changing layer in a direction orthogonal to the array of the ultrasonic resonators by controlling the electrical signal to apply to the shape-changing layer so that the direction of ultrasonic waves is variably changed.

3. The ultrasonic transducer according to claim 1, wherein the ultrasonic waves are variably changed by focusing or diffusing ultrasonic waves or an ultrasonic beam.

4. The ultrasonic transducer according to claim 1, wherein the shape-changing layer has an acoustic velocity having a value that is different from an acoustic velocity of the subject.

5. The ultrasonic transducer according to claim 4, further comprising an acoustic lens which is installed on a subject side of the shape-changing layer and which focuses ultrasonic waves, wherein a lens shape of the acoustic lens changes in association with a change in the shape-changing layer so that the ultrasonic waves are variably focused or diffused.

6. The ultrasonic transducer according to claim 4, wherein the shape-changing layer has a surface on the subject side that is formed into a curved surface shape.

7. The ultrasonic transducer according to claim 1, further comprising an acoustic lens which is installed on the subject side of the shape-changing layer and which focuses an ultrasonic beam at a desired depth.

8. The ultrasonic transducer according to claim 1, wherein the control section comprises:

a first electrode disposed on the ultrasonic resonator side of the shape-changing layer;
a second electrode disposed on the subject side of the shape-changing layer; and
a variable power supply that adjusts an electrical signal to be applied between the first electrode and the second electrode.

9. The ultrasonic transducer according to claim 8, wherein:

the second electrode includes a plurality of electrodes; and
the variable power supply adjusts an electrical signal to be applied between the first electrode and the plural electrodes.

10. The ultrasonic transducer according to claim 1, wherein the shape-changing layer comprises a material, a conductive polymer, an ionic polymer metal composite and a dielectric elastomer, a shape of which changes upon application of a voltage.

11. An ultrasonic transducer comprising:

an ultrasonic resonator array in which ultrasonic resonators for transmitting and receiving ultrasonic waves are arranged one-dimensionally;
a shape-changing layer which is disposed on a subject side of the ultrasonic resonator and a shape of which changes upon receipt of an electrical signal; and
a control section which controls the electrical signal to apply to the shape-changing layer, wherein the control section controls the electrical signal to apply to the shape-changing layer in cooperation with a focus of ultrasonic waves in an aligning direction of the ultrasonic resonators, and also changes the shape of the shape-changing layer in a direction orthogonal to the aligning direction of the ultrasonic resonators so that the ultrasonic waves are variably focused or diffused.

12. An ultrasonic transducer comprising:

an ultrasonic resonator which transmits and receives ultrasonic waves; and
a shape-changing layer which is disposed on a side opposite to a subject side of the ultrasonic resonator and a shape of which changes by an electrical signal, wherein the direction of an ultrasonic beam is variably changed by changing the shape of the shape-changing layer by controlling the electrical signal.

13. The ultrasonic transducer according to claim 12, further comprising:

an ultrasonic resonator array in which ultrasonic resonators for transmitting and receiving ultrasonic waves are arranged; and
a shape-changing layer which is disposed on a side opposite to a subject side of the ultrasonic resonator and a shape of which changes by an electrical signal, wherein by controlling the electrical signal in a direction orthogonal to an aligning direction of the ultrasonic resonator array, the shape-changing layer is changed so that the direction of an ultrasonic beam is variably changed.

14. The ultrasonic transducer according to claim 11, wherein a plurality of electrodes are placed one side of the shape-changing layer a shape of which is changed by an electrical signal so that, by variably changing an electrical signal to apply to the plural electrodes desirably, the shape of the shape-changing layer is changed desirably.

15. An ultrasonic probe comprising:

the ultrasonic transducer according to claim 12; and
a shape-changing layer which is disposed on one portion of the ultrasonic transducer and a shape of which changes by an electrical signal, wherein, by controlling the electrical signal, the shape of the shape-changing layer is changed so as to variably change the direction of the ultrasonic transducer so that the direction of an ultrasonic beam is variably changed.

16. An ultrasonic probe comprising:

the ultrasonic transducer according to claim 12;
a case which encloses the transducer; and
an ultrasonic wave propagating medium which is placed inside the case.

17. The ultrasonic probe according to claim 15, wherein the shape-changing layer is made from a material, selected from the group consisting of a conductive polymer, an ionic polymer metal composite and a dielectric elastomer and a shape of which changes upon application of a voltage.

18. An ultrasonic diagnostic apparatus, which applies a pulse voltage to an ultrasonic resonator so as to transmit ultrasonic waves, and focuses or diffuses received ultrasonic waves, and then converts the waves into an electrical signal so that an ultrasonic diagnostic process is carried out, the ultrasonic diagnostic apparatus comprising:

an ultrasonic resonator array in which ultrasonic resonators which transmit and receive ultrasonic waves are arranged;
a shape-changing layer a shape of which changes upon receipt of an electrical signal; and
a control section which controls the electrical signal to apply to the shape-changing layer, wherein the control section comprises an ultrasonic probe which controls the electrical signal to apply to the shape-changing layer in cooperation with a focus of ultrasonic waves in an aligning direction of the ultrasonic resonators, with the shape of the shape-changing layer changing in a direction orthogonal to the ultrasonic resonator array, so that the ultrasonic waves are variably changed in focus or diffusion.

EP 2 405 671 A1

FIG.1

FIG.2

FIG.3

FIG.4

FIG.5

EP 2 405 671 A1

FIG.6A                    FIG.6B

FIG.7

FIG.8

EP 2 405 671 A1

FIG.9A            FIG.9B            FIG.9C

EP 2 405 671 A1

FIG.10

FIG.11A          FIG.11B          FIG.11C

EP 2 405 671 A1

FIG.12A

FIG.12B

FIG.12C

FIG.13

FIG.14A          FIG.14B          FIG.14C

FIG.15

FIG.16C

FIG.16B

FIG.16A

FIG.17

**INTERNATIONAL SEARCH REPORT**

| International application No. |
| --- |
| PCT/JP2010/001469 |

A.  CLASSIFICATION OF SUBJECT MATTER
*H04R1/34*(2006.01)i, *A61B8/00*(2006.01)i, *H04R17/00*(2006.01)i

According to International Patent Classification (IPC) or to both national classification and IPC

B.   FIELDS SEARCHED

Minimum documentation searched (classification system followed by classification symbols)
H04R1/34, A61B8/00, H04R17/00

Documentation searched other than minimum documentation to the extent that such documents are included in the fields searched
Jitsuyo Shinan Koho          1922-1996    Jitsuyo Shinan Toroku Koho    1996-2010
Kokai Jitsuyo Shinan Koho    1971-2010    Toroku Jitsuyo Shinan Koho    1994-2010

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
| --- | --- | --- |
| X | WO 2008/068932 A1  (Okusonic Corp.),<br>12 June 2008 (12.06.2008),<br>paragraphs [0029] to [0058]; fig. 1 to 8<br>(Family: none) | 1-3,7,18 |
| X<br>Y | JP 04-152940 A  (Olympus Optical Co., Ltd.),<br>26 May 1992 (26.05.1992),<br>page 4, upper right column, line 15 to lower<br>right column, line 10; fig. 5, 6<br>(Family: none) | 4,6,8-10<br>5,11,14 |
| Y | JP 63-177700 A  (Omron Tateisi Electronics Co.),<br>21 July 1988 (21.07.1988),<br>page 2, upper right column, line 2 to page 3,<br>upper left column, line 17; fig. 1, 2<br>& US 4880012 A | 5,11,14 |

☒  Further documents are listed in the continuation of Box C.        ☐   See patent family annex.

*    Special categories of cited documents:
"A"  document defining the general state of the art which is not considered to be of particular relevance
"E"  earlier application or patent but published on or after the international filing date
"L"  document which may throw doubts on priority claim(s) or which is cited to establish the publication date of another citation or other special reason (as specified)
"O"  document referring to an oral disclosure, use, exhibition or other means
"P"  document published prior to the international filing date but later than the priority date claimed

"T"  later document published after the international filing date or priority date and not in conflict with the application but cited to understand the principle or theory underlying the invention
"X"  document of particular relevance; the claimed invention cannot be considered novel or cannot be considered to involve an inventive step when the document is taken alone
"Y"  document of particular relevance; the claimed invention cannot be considered to involve an inventive step when the document is combined with one or more other such documents, such combination being obvious to a person skilled in the art
"&"  document member of the same patent family

| Date of the actual completion of the international search<br>10 June, 2010 (10.06.10) | Date of mailing of the international search report<br>22 June, 2010 (22.06.10) |
| --- | --- |
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2010/001469

C (Continuation). DOCUMENTS CONSIDERED TO BE RELEVANT

| Category* | Citation of document, with indication, where appropriate, of the relevant passages | Relevant to claim No. |
|---|---|---|
| X | JP 02-093362 A (Toshiba Corp.), 04 April 1990 (04.04.1990), page 2, lower right column, line 15 to page 3, lower right column, line 2; fig. 1 to 4 (Family: none) | 12,13,15-17 |

Form PCT/ISA/210 (continuation of second sheet) (July 2009)

**INTERNATIONAL SEARCH REPORT**

International application No.

PCT/JP2010/001469

| Box No. II | Observations where certain claims were found unsearchable (Continuation of item 2 of first sheet) |
|---|---|

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |
|---|---|

This International Searching Authority found multiple inventions in this international application, as follows:

This international application contains seven inventions which do not comply with the requirement of unity of invention for the following reason.
Document 1 describes an ultrasonic probe comprising multiple ultrasonic vibrators arranged linearly and an acoustic lens provided on the subject side of the ultrasonic vibrators, wherein the ultrasonic probe is provided with an electrode for applying an electrical signal to the acoustic lens, and the direction of a beam is varied by changing the shape of the acoustic lens in the direction orthogonal to the direction of arrangement of the ultrasonic vibrators by controlling the voltage to be applied through the electrode. Therefore, the inventions in claims 1, 2 (Continued to the extra sheet.)

1. ☒ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (July 2009)

**INTERNATIONAL SEARCH REPORT**

International application No.

PCT/JP2010/001469

Continuation of Box No.III of continuation of first sheet(2)

are not considered to be novel over the invention described in document 1, and they have no special technical feature.

As a result of the investigation of special technical features of claims dependent on claim 1 at the time of issuance of an order for payment of additional fees, five groups of inventions linked by the following respective special technical features are considered to be contained.

Note that the inventions in claims 1, 2 which have no special technical feature are classified as invention 1.

(Invention 1) The inventions in claims 1-3, 18

An ultrasonic transducer having a configuration "being provided with an ultrasonic vibrator which transmits and receives ultrasonic waves, a shape change layer which is provided on the subject side of the ultrasonic vibrator and the shape of which is changed upon receiving an electrical signal, and a control means which controls the electrical signal to be applied to the shape change layer, wherein the control means varies the direction of the ultrasonic waves by changing the shape of the shape change layer by controlling the electrical signal to be applied to the shape change layer" (hereinafter referred to as feature A), and a configuration being provided with "an ultrasonic vibrator array in which a plurality of ultrasonic vibrators for transmitting and receiving ultrasonic waves are arranged", "wherein the shape of the shape change layer is changed in the direction orthogonal to the ultrasonic vibrator array".

Note that since the inventions in claims 3, 18 are constituted by the mere addition of well-known art to the invention in claim 1 and do not provide a novel effect, they are classified as invention 1.

(Invention 2) The inventions in claims 4-6

An ultrasonic transducer comprising feature A and a configuration that "the sonic speed of the shape change layer has a value different from that of the sonic speed of the subject".

(Invention 3) The invention in claim 7

An ultrasonic transducer comprising feature A and a configuration "provided with an acoustic lens for converging an ultrasonic beam in an arbitrary depth on the subject side of the shape change layer".

(Invention 4) The inventions in claims 8, 9

An ultrasonic transducer comprising feature A and a configuration that "the control means is provided with a first electrode which is disposed on the ultrasonic vibrator side of the shape change layer, a second electrode which is disposed on the subject side of the shape change layer, and a variable power source which adjusts the electrical signal to be applied between the first electrode and the second electrode".

(Invention 5) The invention in claim 10

An ultrasonic transducer comprising feature A and a configuration that "the shape change layer is conductive polymeric, ion conductive polymeric, and dielectric elastomeric materials, the shapes of which are changed by applying voltage".

The invention in claims 11 and the invention in claim 12, which are independent claims, are not regarded as including all of the matter to define the invention of the invention in claim 2, and thus each do not have a technical relationship involving one or more of the same or corresponding special technical features with the invention in claim 1. Therefore, they are not considered to be so linked as to form a single general inventive concept.

(Invention 6) The inventions in claims 11, 14

(Invention 7) The inventions in claims 12, 13, 15-17

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003047084 A
- JP 2093362 A
- JP 2001003776 A
- JP 2003075036 A
- JP 2007152101 A
- US 06238346 B
- JP 2009051314 A
- JP 2009051315 A
- JP 2009054551 A